# EUROPEAN PATENT SPECIFICATION

(11) **EP 4 412 566 B1**
(45) Date of publication and mention of the grant of the patent: **27.05.2026**
(21) Application number: 22799898.6
(22) Date of filing: 30.09.2022
(51) Int. Cl.: A61F 13/00, A61K 9/70

(54) **WOUND DRESSING APPARATUSES FOR NITRIC OXIDE DELIVERY**
WUNDVERBANDVORRICHTUNGEN ZUR STICKOXIDFREISETZUNG
APPAREILS DE PANSEMENT POUR L'ADMINISTRATION D'OXYDE NITRIQUE

(30) Priority: 06.10.2021 GB 202114294
(43) Date of publication of application: 14.08.2024
(73) Proprietor: T.J.Smith and Nephew,Limited, Hull HU3 2BN (GB)
(72) Inventor: BROWNHILL, Varuni, Rachindra, Hull HU3 2BN (GB); DAGGER, Anthony, Colin, Hull HU3 2BN (GB); EARL, Michael, Hull HU3 2BN (GB); ELLERINGTON, Mark, John, Hull HU3 2BN (GB); FITZGERALD, Daniel, James, Hull HU3 2BN (GB); FRY, Nicholas, Charlton, Hull HU3 2BN (GB); GOWANS, Philip, Hull HU3 2BN (GB); HAMMOND, Victoria, Jody, Hull HU3 2BN (GB); KOTECHA, Bindiya, Hull HU3 2BN (GB); LECOMTE, Helene, Anne, Hull HU3 2BN (GB); MIDDLETON, Natasha Rose, Hull HU3 2BN (GB)
(74) Representative: Appleyard Lees IP LLP
(86) International application number: PCT/EP2022/077402
(87) International publication number: WO 2023/057356

(56) References cited:
- WO-A1-2014/188174
- WO-A1-2017/089831
- WO-A1-2020/256843
- US-A1- 2011 160 686
- US-A1- 2016 158 066
- US-B2- 7 230 154

## Description

### BACKGROUND

### Technical Field

Disclosed herein are materials, devices, methods, and systems, such as therapeutic compositions, wound care materials, their uses, and methods of treatment therewith. In some examples, the materials, devices, and systems described herein comprise wound dressings, wound dressing components and methods of making the same configured for nitric oxide (NO) delivery and/or the delivery of other actives.

### Description of the Related Art

Nitric oxide (NO) is a well-known molecule with multiple biological functions. For example, nitric oxide influences blood vessel vasodilation, stimulates angiogenesis, influences the host immune response, and demonstrates potent, broad spectrum antimicrobial activity and anti-biofilm activity. Due to these multiple roles, NO demonstrates a potent effect on tissue and increased amounts of NO may support the acceleration of healing in wounds, particularly chronic wounds.

Additionally, diabetic patients often have lower levels of nitric oxide as compared to healthy patients, and diminished supply of nitric oxide in diabetic patients is a compounding factor in a healing chronic ulcer. Diminished supply of nitric oxide may lead to vascular damage, such as endothelial dysfunction and vascular inflammation. Vascular damage may also lead to decreased blood flow to the extremities, thereby potentially causing the diabetic patient to be more likely to develop neuropathy and non-healing ulcers, and to be at a greater risk for lower limb amputation.

Consequently, there is a need for improved mechanisms of delivering an effective dose of nitric oxide to a wound. Under normal conditions, nitric oxide (NO), a free radical, is short-lived and converted to a more stable chemical species within seconds of production. Thus, for example, if gaseous nitric oxide contacts air, the gaseous nitric oxide will be rapidly oxidized to generate nitrogen dioxide (NO₂). Accordingly, it may be difficult to maintain high concentrations of nitric oxide within a wound dressing or other similar structure for a prolonged period of time. Therefore, a device or a wound dressing having one or more layers containing more stable compositions may effectively generate nitric oxide over time upon activation, for the stable and sustained delivery of nitric oxide to biological tissues. Of particular interest are mechanisms of delivering nitric oxide in combination with use of a wound dressing, particularly a negative pressure wound dressing and/or while undergoing negative pressure wound therapy and/or other appropriate therapies.

US2011/160686A1 relates to a wound dressing having absorbability and improved so as not to be adhere to wounds, which includes a perforated material having plural through-holes, and an absorbent material, to allow fluid penetration from the bottom face side of the perforated material into the inside of the absorbent material.

WO2020/256843A1 relates to a citric acid containing dressing for treating a tissue site with negative pressure comprising a cover, a manifold, and a perforated polymer film and/or a sealing layer, and citric acid may be present on or incorporated in, for example, the perforated polymer film and/or the sealing layer.

WO2017/089831A1 relates to skin dressings that are useful in the treatment of conditions associated with tissue ischaemia and skin lesions including those that are infected, such as burns and surgical wounds and chronic wounds which may effect transdermal delivery of pharmaceutically active agents.

WO2014/188174A1 relates to a system comprising: (i) a layer containing a nitrite; and (ii) a hydrogel that contains hydrogen ions, for the treatment of a condition associated with tissue ischaemia or a wound.

US2004/126413A1 relates to a wound dressing including an absorbent core defining opposed proximal and distal surfaces, a perforated skin adherent facing layer secured to a proximal surface of the absorbent core and a liquid impervious, vapor permeable backing layer connected to a distal surface of the absorbent core; the backing layer defining at least one compliant element disassociated from the distal surface of the absorbent core and including at least one ridge extending outwardly relative to the distal surface of the absorbent core.

US2016/158066A1 relates to a negative pressure wound dressing including a backing layer, a water blocking layer, an absorbent layer and a wound contacting layer; wherein, the backing layer includes a connecting aperture for fluidly communicating to a negative pressure source.

### SUMMARY

Embodiments of the present disclosure relate to materials, devices, methods, and systems for wound treatment. Some disclosed embodiments relate to materials, devices, methods, and systems for delivering nitric oxide to a wound. It will be understood by one of skill in the art that application of the materials, devices, methods, and systems described herein are not limited to a particular tissue or a particular injury.

A wound dressing apparatus is provided as set out in the appended claims.

The wound dressing for treating a wound may comprise a cover layer configured to form a seal around a wound, an activator layer, a dry nitric oxide source layer, the dry nitric oxide source layer free or relatively free of liquid, and an acquisition distribution layer.

The wound dressing may further comprise a masking layer, the masking layer configured to at least partially limit visualization of the wound. The dry nitric oxide source layer may comprise a nitrite salt. The nitrite salt may comprise sodium nitrite. The activator layer may be positioned above the nitric oxide source layer. The nitric oxide source layer may be positioned above the activator layer. The acquisition distribution layer may be positioned between the activator layer and the dry nitric oxide source layer. The activator layer may comprise a hydrogel or a xerogel. The wound dressing may comprise a second dry nitric oxide source layer. The wound dressing may be configured to generate nitric oxide when the wound dressing is placed over a wound. The wound dressing may be configured to not generate nitric oxide prior to placement over a wound.

Alternative or additional embodiments described herein provide a composition comprising one or more of the features of the foregoing description or of any description elsewhere herein.

Alternative or additional embodiments described herein provide a wound contact layer comprising one or more of the features of the foregoing description or of any description elsewhere herein.

Alternative or additional embodiments described herein provide a wound dressing comprising one or more of the features of the foregoing description or of any description elsewhere herein.

Alternative or additional embodiments described herein provide a wound treatment system comprising one or more of the features of the foregoing description or of any description elsewhere herein.

Alternative or additional embodiments described herein provide a method of treating a wound comprising one or more of the features of the foregoing description or of any description elsewhere herein.

The wound dressing apparatus comprises an absorbent layer having a wound-facing surface and an opposite, non-wounding surface and a gel material adhered to the wound-facing surface of the absorbent layer. The gel material comprises a cured patterned structure defining a plurality of fluid channels extending therethrough.

The gel material comprises acidic groups or moieties.

The wound dressing apparatus further comprises a cover layer positioned above the absorbent layer.

In some embodiments, the wound dressing apparatus may further comprise a tissue interface layer below the gel material and adhered to a peripheral portion of the cover layer. The tissue interface layer comprises a central opening positioned beneath at least a portion of the gel material.

In some embodiments, the tissue interface layer may comprise silicone.

The wound dressing apparatus further comprises one or more nitrite providing layers.

The one or more nitrite providing layers is positioned beneath the cover layer.

In some embodiments, the one or more nitrite providing layers may be positioned beneath the gel material.

In some embodiments, the one or more nitrite providing layers may be positioned between the cover layer and the absorbent layer.

In some embodiments, the wound dressing apparatus may further comprise an acquisition distribution layer. The acquisition distribution layer may be a composite material comprising a gel material comprising acidic groups or moieties. The acquisition distribution layer may be provided between the one or more nitrite providing layers and the gel material. The acquisition distribution layer may be provided between a cover layer and the absorbent layer.

In some embodiments, the gel material adhered to the wound-facing surface of the absorbent layer may be configured to provide acid to the one or more nitrite providing layers to release nitric oxide.

In some embodiments, the absorbent layer may comprise foam.

In some embodiments, the gel material may be provided on a mesh.

In some embodiments, the cured patterned structure may be molded.

In some embodiments, the absorbent layer may be perforated.

In some embodiments, the gel material may be at least partially saturated into the absorbent layer.

In some embodiments, the perforated absorbent layer may be saturated with the gel material and further comprise a second gel material adhered to the perforated absorbent layer.

In some embodiments, the cured pattern structure may have a smaller outer perimeter than an outer perimeter of the absorbent layer.

In an aspect of the disclosure, a method of making a wound dressing material may comprise providing an absorbent layer having a wound-facing surface and an opposite, non-wound facing surface and adhering a gel material to the wound facing surface of the absorbent layer. The gel material may be cured to form a patterned structure defining a plurality of fluid channels extending therethrough.

The gel material may comprise acidic groups or moieties.

The gel material may be formed in a patterned mold.

The gel material may be spread across the wound-facing surface of the absorbent layer and partially cured before applying the patterned mold.

The gel material may be filled into the patterned mold and be at least partially cured before applying the absorbent layer to the gel material.

The method may further comprise applying a force to the foam to adhere the foam to the gel material.

A weight may be applied to the mold.

The gel material may be cured using heat.

The gel material may be cured using UV light.

The method may further comprise at least partially permeating the gel material into at least a portion of the absorbent layer prior to curing.

The method may further comprise wetting a perforated mesh material with the gel material before adhering the gel material to the absorbent layer.

The method may further comprise adhering the gel material to a perforated absorbent layer.

### BRIEF DESCRIPTION OF THE DRAWINGS

FIG. 1 is a schematic diagram of an example of a negative pressure wound therapy system;
FIG. 2A illustrates an embodiment of a negative pressure wound treatment system employing a pump, a flexible fluidic connector and a wound dressing capable of absorbing and storing wound exudate;
FIG. 2B illustrates an embodiment of a negative pressure wound treatment system employing a flexible fluidic connector and a wound dressing capable of absorbing and storing wound exudate;
FIG. 2C illustrates a cross section of an embodiment of a fluidic connector connected to a wound dressing;
FIG. 2D illustrates a cross-section of an embodiment of a wound dressing;
FIGS. 3A-3D illustrate embodiments of wound dressings capable of absorbing and storing wound exudate to be used without negative pressure;
FIG. 3E illustrates a cross section of an embodiment of a wound dressing capable of absorbing and storing wound exudate to be used without negative pressure;
FIG. 4 is an exploded view of an embodiment of a wound dressing which can generate nitric oxide;
FIG. 5 is a cross sectional view of the wound dressing of FIG. 4;
FIG. 6 illustrates an example of a chemiluminescence experimental protocol equipment setup;
FIGS. 7A-B illustrates a negative pressure and nitric oxide delivery experiment;
FIG. 8A depicts an example of chemiluminescence experimental results for a sodium nitrate mesh;
FIG. 8B depicts an example of chemiluminescence experimental results for a full dressing design with a pull-out tab and self-sealing borders;
FIG. 8C depicts an example of chemiluminescence experimental results for a dressing containing a degradable film;
FIG. 9 depicts an example of a graph displaying peak NO and NO₂ outputs for acrylic adhesive containing hydrogels;
FIGS. 10A-D depict examples of chemiluminescence experimental results for nitric oxide dressing;
FIGS. 11A-D depicts embodiments of a wound dressing configured to generate nitric oxide;
FIG. 12A depicts a cross-sectional view of a wound dressing including a cover layer, an absorbent layer, and a gel layer having a gel material cured to a perforated mesh;
FIG. 12B depicts a bottom perspective view of a wound dressing component including an absorbent layer and a perforated gel layer adhered to the absorbent layer;
FIG. 12C depicts a cross-sectional view of a wound dressing including a cover layer, an absorbent layer, and a perforated gel layer adhered to the absorbent layer;
FIG. 12D depicts a cross-sectional view of a wound dressing including a cover layer, an absorbent layer, a perforated absorbent layer, a perforated gel layer adhered to the perforated absorbent layer, and a tissue interface layer;
FIG. 12E depicts a bottom perspective view of a wound dressing component including an absorbent layer, a perforated absorbent layer, and a perforated gel layer adhered to the perforated absorbent layer;
FIG. 12F depicts a cross-sectional view of a wound dressing including a cover layer, an absorbent layer, a gel permeated perforated absorbent layer, a perforated gel layer adhered to the gel permeated perforated absorbent layer, and a tissue interface layer;
FIG. 12G depicts a cross-sectional view of a wound dressing including a cover layer, an absorbent layer, a gel layer adhered to only a portion of the absorbent layer, and a tissue interface layer;
FIG. 12H depicts a perspective view of three dimensional gel formations having gaps or spacings in between that act as fluid channels;
FIG. 12I depicts a wound dressing including a cover layer, an absorbent layer, a perforated absorbent layer, a gel layer adhered to only a portion of the perforated absorbent layer, and a tissue interface layer.

### DETAILED DESCRIPTION

### Overview

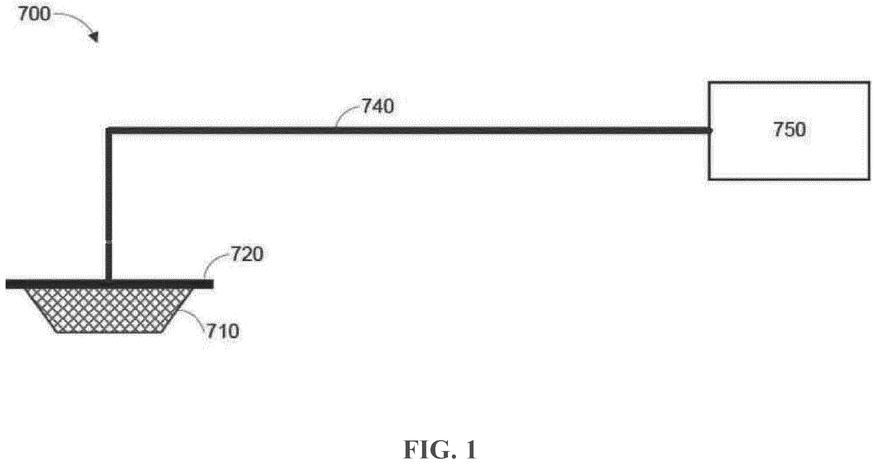

Aspects of the disclosure described herein relate to materials, apparatuses, methods, and systems that incorporate, or comprise, or utilize one or more compositions and/or materials that effectively generate gases (e.g. nitric oxide) over time upon activation. Embodiments herein may be directed toward a device and/or a wound dressing having one or more layers containing compositions and/or materials that effectively generate nitric oxide over time upon activation. For example, one or more nitric oxide generating layers may include a nitrite delivery layer which contains nitrite salts and can release nitrite ions, such that the nitrite ions can generate nitric oxide upon reaction with acids. In some embodiments, the one or more nitric oxide generating layers can further include an acidic-group-providing layer in addition to the nitrite delivery layer. The one or more nitric oxide generating layers may be utilized as a stand-alone component for separately positioning at a wound site, or may be incorporated into any number of multi-layer wound dressings and wound treatment apparatuses, such as described herein below with respect to Figures 1 through 11D. Embodiments of the present disclosure are generally applicable to use under ambient conditions, in negative pressure or reduced pressure therapy systems, or in compression therapy systems.

Some of the preferred embodiments described herein incorporate, or comprise, or utilize one or more nitric oxide generating layers. Such one or more nitric oxide generating layers may possess one or more of the following functional features: inflammation-related activities, blood flow-related activities, antimicrobial, anti-planktonic and anti-biofilm activities, ease of application or/and removal as one piece, cuttability/tearability, conformability to the three-dimensional contour of a wound surface, durability to wear, compatibility with negative pressure wound therapy or/and compression wound therapy, exudate management, capability of facilitating autolytic debridement of wounds, capability of promoting wound healing, and self-indication of compositional or functional changes. The antimicrobial activities, such as *in vitro* antimicrobial activities, can include one or more of the following: broad-spectrum antimicrobial activity, anti-biofilm activity, rapid speed of kill against microorganisms, sustained kill against microorganisms; and the microorganisms can include one or more of the following: Gram-negative bacteria, Gram-positive bacteria, fungi, yeasts, viruses, algae, archaea and protozoa.

Certain preferred embodiments described herein provide a wound treatment system. Such a wound treatment system may comprise nitric oxide generating layers, configured to be sized for positioning over a wound and/or the periwound area. One of skill in the art will understand that when an apparatus/dressing/layer is described as being placed on or over a wound, such an apparatus/dressing/layer may extend over and treat the periwound area. In some instances, stimulation of the periwound area and/or the wound edge may play a role in initiating the wound healing process, and the wound healing process can be activated through the delivery of nitric oxide to the periwound area and/or the wound edge. The delivery of nitric oxide to the periwound area and/or the wound edge may target, for example epithelial cell activity to promote migration of epithelial tongue; vasodilation of the microcirculation in the skin surrounding the wound to promote profusion by providing oxygen and nutrients; and neo-angiogenesis to promote granulation tissue formation. The wound treatment systems described herein may further comprise a secondary wound dressing configured to be separately positioned over the nitric oxide generating layers. The nitric oxide generating layers may have an adhesive adhered to the lower surface; and the adhesive can be configured such that the nitric oxide generating layers may be placed in proximity to the wound. The secondary wound dressing, if used, may adhere to skin surrounding the wound and may have the same size or may be larger than the nitric oxide generating layers, such that the nitric oxide generating layers will touch or be placed in proximity to the wound and/or the periwound area. The secondary wound dressing can be alternatively or additionally configured to form a seal to skin surrounding the wound so that the nitric oxide generating layers will touch or be placed in proximity to the wound. The wound treatment system may further comprise a source of negative pressure configured to supply negative pressure through the secondary wound dressing and through the wound contact layer to the wound.

Certain other preferred embodiments described herein provide a multi-layered wound dressing, such as described herein the specification with respect to FIGS. 1 through 11D. Such a multi-layered wound dressing may incorporate the one or more nitric oxide generating layers as component layers thereof or, alternatively, may comprise a composite or laminate including the one or more nitric oxide generating layers as part of one of the component layers thereof. The multi-layered wound dressing may comprise: nitric oxide generating layers as described above or described elsewhere herein; a transmission layer and/or absorbent layer over/under the one or more nitric oxide generating layers; a wound contact layer under the one or more nitric oxide generating layers; and a cover layer over the transmission layer and/or absorbent layer. The wound dressing may further comprise a negative pressure port positioned on or above the cover layer. The one or more nitric oxide generating layers may have a perimeter shape that is substantially the same as a perimeter shape of the cover layer. Alternatively, the one or more nitric oxide generating layers may have a perimeter shape that is smaller than a perimeter shape of the cover layer.

One of skill in the art will understand that nitric oxide generating compositions, such as any disclosed herein this "Overview" section or elsewhere in the specification, may be loaded within the one or more nitric oxide generating layers in any suitable form, such as via adsorption, absorption, chemical and/or physical attachment entanglement, and/or via powder form. One of skill in the art will further understand that reactive compositions, such as any disclosed herein this section or elsewhere in the specification may be incorporated into any suitable absorbent layer disclosed herein this section or elsewhere in the specification by any suitable means, and/or any suitable transmission layer disclosed herein this section or elsewhere in the specification, and/or any foam layer disclosed herein this section or elsewhere in the specification.

The wound treatment systems and multi-layered wound dressings disclosed above or disclosed elsewhere herein the specification incorporate or comprise one or more nitric oxide generating layers. As described herein this section or elsewhere in the specification, particularly below, the one or more nitric oxide generating layers may be configured to be activated to release nitric oxide. At least a portion of the released nitric oxide may be released, for example by diffusion. To facilitate release and diffusion of nitric oxide, the nitric oxide generating layers may be placed proximate to the wound.

An aspect of the disclosure described herein provides a method to treat a wound, intact tissue, or other suitable location. Such a method may include placing nitric oxide generating layers, either separately or by placing a multi-layered wound dressing having nitric oxide generating layers, over the wound. The method may comprise adhering the separate nitric oxide generating layers and/or the multi-layer wound dressing having nitric oxide generating layers to healthy skin around the wound. Such a method may further comprise one or more of the following steps: A further wound dressing can be placed over the separate nitric oxide generating layers or multi-layered wound dressing having the nitric oxide generating layers that is placed over the wound. Wound exudate, or any moist or aqueous medium other than wound exudate, may be provided to reach and/or touch the nitric oxide generating layers. Wound exudate, or any moist or aqueous medium other than wound exudate may be diffused or wicked into the wound dressing incorporating the nitric oxide generating layers or into a wound dressing provided over the nitric oxide generating layers. Negative pressure may be applied to the separate nitric oxide generating layers or multi-layered wound dressing having the nitric oxide generating layers, such that wound exudate is suctioned into the nitric oxide generating layers directly, or into the wound dressing incorporating the nitric oxide generating layers, or into a wound dressing provided over the nitric oxide generating layers.

One of skill in the art will understand that wound dressings, devices and systems disclosed herein this "Overview" section or elsewhere in the specification may include one or more layers, compositions, materials or components that generate gases other than nitric oxide in addition to or in place of the nitric oxide generating layers, compositions or materials. For example, a wound dressing or a device can include one or more layers that effectively generate vasodilatory agents, such as carbon monoxide or hydrogen sulfide, over time upon activation.

One of skill in the art will further understand that carbon monoxide and/or hydrogen sulfide may be used in place of a nitric oxide delivery element (such as a layer) or in combination with a nitric oxide delivery element (such as a layer) where suitable. Further details regarding generation and delivery of carbon monoxide and/or hydrogen sulfide may be found in chapter six of the text Inorganic and Organometallic Transition Metal Complexes with Biological Molecules and Living Cells, ISBN 978-0-12-803814-7. For example, hydrogen sulfide may be generated from elements/layers that contain cleavable/releasable hydrogen sulfide, diallyl thiosulfinate, GYY4137, S-Mesalamine ATB-429, S-Naproxen ATB-346, S-Diclofenac ATB-337/ACS-15. For example, carbon monoxide may be generated from elements/layers that provide of complexes of carbon monoxide bound to suitable metals such as chromium, molybdenum, tungsten, manganese, rhenium, iron, ruthenium, cobalt, rhodium, and iridium. Such complexes may be enzymatically triggered to release carbon monoxide, photo-cleavable, and/or responsive to interaction with a suitable ligand to induce release of carbon monoxide.

### Methods of Treating a Wound

The wound dressing apparatus described herein may be of use in a method of treating a wound, intact tissue, or other suitable location. Such a method may include placing one or more nitric oxide generating layers, either separately or by placing a multi-layered wound dressing having one or more nitric oxide generating layers over the wound. The method may comprise adhering the separate one or more nitric oxide generating layers and/or the multi-layer wound dressing having one or more nitric oxide generating layers to healthy skin around the wound, such as the periwound area. The method may further comprise one or more of the following steps: A further wound dressing can be placed over the separate one or more nitric oxide generating layers or multi-layered wound dressing having the one or more nitric oxide generating layers that is placed over the wound. Wound exudate, or any moist or aqueous medium other than wound exudate, may be provided to reach and/or touch the one or more nitric oxide generating layers. Wound exudate, or any moist or aqueous medium other than wound exudate may be diffused or wicked into the wound dressing incorporating the one or more nitric oxide generating layers or into a wound dressing provided over the one or more nitric oxide generating layers. Negative pressure may be applied to the separate one or more nitric oxide generating layers or multi-layered wound dressing having the one or more nitric oxide generating layers, as described in the following "Negative Pressure Wound Therapy (NPWT) Systems" section or described elsewhere herein the specification, such that wound exudate is suctioned into the one or more nitric oxide generating layers directly, or into the wound dressing incorporating the one or more nitric oxide generating layers, or into a wound dressing provided over the one or more nitric oxide generating layers.

The method of treating a wound, intact tissue, or other suitable location as described above or described elsewhere herein may further comprise delivering negative pressure through the wound contact layer to the wound, as described in the following "Negative Pressure Wound Therapy (NPWT) Systems" section or described elsewhere herein the specification. The wound contact layer may substantially maintain the negative pressure delivered for at least about 24 hours, or for at least about 48 hours, or for at least about 72 hours. Alternatively, the method of treating a wound, intact tissue, or other suitable location may comprise applying compression (positive) pressure through the wound contact layer to the wound. Alternatively, the method may comprise altering ambient pressure, negative pressure and compression pressure in a programmable manner through the wound contact layer to the wound.

The method of treating a wound, intact tissue, or other suitable location may comprise using the wound contact layer, or the wound treatment system or wound dressing that comprises the wound contact layer, under ambient conditions not in connection with a negative pressure wound therapy system as described above, or described elsewhere herein.

The method of treating a wound, intact tissue, or other suitable location may reduce the wound bioburden, for example, at least *in vitro,* by reducing the numbers (CFU/sample) of viable microorganisms within the first 4 hours after the application wound contact layer. In some examples, the numbers of viable microorganisms may be reduced by four log or more, 48 to 72 hours after positioning the wound dressing in contact with the microorganisms.

### Negative Pressure Wound Therapy (NPWT) Systems

It will be understood that embodiments of the present disclosure are generally applicable to, but not limited to, use in topical negative pressure ("TNP") therapy systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). In addition, the therapy allows for less disturbance of a wound leading to more rapid healing. TNP therapy systems may also assist on the healing of surgically closed wounds by removing fluid and by helping to stabilize the tissue in the apposed position of closure. A further beneficial use of TNP therapy can be found in grafts and flaps where removal of excess fluid is important and close proximity of the graft to tissue is required in order to ensure tissue viability.

As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (e.g., - 40 mmHg is less than -60 mmHg). Negative pressure that is "more" or "greater" than -X mmHg corresponds to pressure that is further from atmospheric pressure (e.g., -80 mmHg is more than - 60 mmHg). In some embodiments, local ambient atmospheric pressure is used as a reference point, and such local atmospheric pressure may not necessarily be, for example, 760 mmHg.

The negative pressure range for some embodiments of the present disclosure can be approximately -80 mmHg, or between about -20 mmHg and -200 mmHg. Note that these pressures are relative to normal ambient atmospheric pressure, which can be 760 mmHg. Thus, - 200 mmHg would be about 560 mmHg in practical terms. In some embodiments, the pressure range can be between about -40 mmHg and -150 mmHg. Alternatively, a pressure range of up to -75 mmHg, up to -80 mmHg or over -80 mmHg can be used. Also in other embodiments a pressure range of below -75 mmHg can be used. Alternatively, a pressure range of over approximately - 100 mmHg, or even -150 mmHg, can be supplied by the negative pressure apparatus.

In some embodiments of wound closure devices described herein, increased wound contraction can lead to increased tissue expansion in the surrounding wound tissue. This effect may be increased by varying the force applied to the tissue, for example by varying the negative pressure applied to the wound over time, possibly in conjunction with increased tensile forces applied to the wound via embodiments of the wound closure devices. In some embodiments, negative pressure may be varied over time for example using a sinusoidal wave, square wave, or in synchronization with one or more patient physiological indices (e.g., heartbeat). Examples of such applications where additional disclosure relating to the preceding may be found include U.S. Patent No. 8,235,955, titled "Wound treatment apparatus and method," issued on August 7, 2012; and U.S. Patent No. 7,753,894, titled "Wound cleansing apparatus with stress," issued July 13, 2010.

The wound dressings, wound dressing components, wound treatment apparatuses and methods described herein may also be used in combination or in addition to those described in International Application No. PCT/IB2013/001469, filed May 22, 2013, published as WO 2013/175306 A2 on November 28, 2013, titled "APPARATUSES AND METHODS FOR NEGATIVE PRESSURE WOUND THERAPY," International Application No. PCT/IB2013/002060, filed on July 31, 2013, published as WO2014/020440, entitled "WOUND DRESSING,". The wound dressings, wound treatment apparatuses and methods described herein may also be used in combination or in addition to those described in US Patent No. 9,061,095, titled "WOUND DRESSING AND METHOD OF USE," issued on June 23, 2015; and U.S. Application Publication No. 2016/0339158, titled "FLUIDIC CONNECTOR FOR NEGATIVE PRESSURE WOUND THERAPY," published on November 24, 2016, including further details relating to embodiments of wound dressings, the wound dressing components and principles, and the materials used for the wound dressings.

Additionally, some embodiments related to TNP wound treatment comprising a wound dressing in combination with a pump or associated electronics described herein may also be used in combination or in addition to those described in International Publication No. WO 2016/174048 A1, entitled "REDUCED PRESSURE APPARATUSES", published on November 3, 2016. The pump or associate electronic components may be integrated into the wound dressing to provide a single article to be applied to the wound.

### Multi-Layered Wound Dressings for NPWT

Figure 1 illustrates an example of a negative pressure wound therapy system 700. The system includes a wound cavity 710 covered by a wound dressing 720, which can be a dressing according to any of the examples described herein. The dressing 720 can be positioned on, inside, over, or around the wound cavity 710 and further seal the wound cavity so that negative pressure can be maintained in the wound cavity. For example, a film layer of the wound dressing 720 can provide substantially fluid impermeable seal over the wound cavity 710. In some embodiments, a wound filler, such as a layer of foam or gauze, may be utilized to pack the wound. The wound filler may include one or more nitric oxide generating layers (e.g. a nitrite delivery layer, an acidic-group providing layer) as described herein this section or elsewhere in the specification. For example, in a traditional negative pressure wound therapy system utilizing foam or gauze, such as the Smith & Nephew RENASYS Negative Pressure Wound Therapy System utilizing foam (RENASYS-F) or gauze (RENASYS-G), the foam or gauze may be supplemented with nitric oxide generating layers as described above. When supplementing a foam or gauze layer or other wound packing material, the one or more nitric oxide generating layers may either be separately inserted into the wound or may be pre-attached with the wound packing material for insertion into the wound.

A single or multi lumen tube or conduit 740 connects the wound dressing 720 with a negative pressure device 750 configured to supply reduced pressure. The negative pressure device 750 includes a negative pressure source. The negative pressure device 750 can be a canisterless device (meaning that exudate is collected in the wound dressing and/or is transferred via the tube 740 for collection to another location). In some embodiments, the negative pressure device 750 can be configured to include or support a canister. Additionally, in any of the embodiments disclosed herein, the negative pressure device 750 can be fully or partially embedded in, mounted to, or supported by the wound dressing 720.

The conduit 740 can be any suitable article configured to provide at least a substantially sealed fluid flow path or pathway between the negative pressure device 750 and the wound cavity 710 so as to supply reduced pressure to the wound cavity. The conduit 740 can be formed from polyurethane, PVC, nylon, polyethylene, silicone, or any other suitable rigid or flexible material. In some embodiments, the wound dressing 720 can have a port configured to receive an end of the conduit 740. For example, a port can include a hole in the film layer. In some embodiments, the conduit 740 can otherwise pass through and/or under a film layer of the wound dressing 720 to supply reduced pressure to the wound cavity 710 so as to maintain a desired level of reduced pressure in the wound cavity. In some embodiments, at least a part of the conduit 740 is integral with or attached to the wound dressing 720.

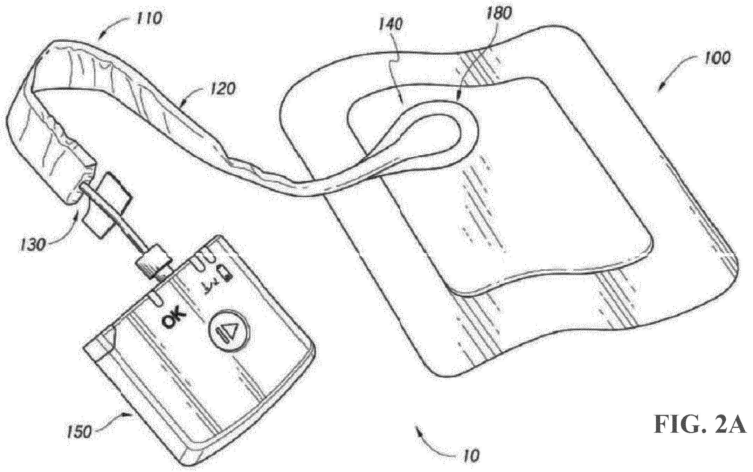

Figure 2A illustrates an embodiment of a negative pressure wound treatment system 10 employing a wound dressing 100 in conjunction with a fluidic connector 110. Additional examples related to negative pressure wound treatment comprising a wound dressing in combination with a pump as described herein may also be used in combination or in addition to those described in US Patent No. 9,061,095. Here, the fluidic connector 110 may comprise an elongate conduit, more preferably a bridge 120 having a proximal end 130 and a distal end 140, and an applicator 180 at the distal end 140 of the bridge 120. The system 10 may include a source of negative pressure such as a pump or negative pressure unit 150 capable of supplying negative pressure. The pump may comprise a canister or other container for the storage of wound exudates and other fluids that may be removed from the wound. A canister or container may also be provided separate from the pump. In some embodiments, the pump 150 can be a canisterless pump such as the PICO^{™} pump, as sold by Smith & Nephew. The pump 150 may be connected to the bridge 120 via a tube, or the pump 150 may be connected directly to the bridge 120. In use, the dressing 100 is placed over a suitably-prepared wound, which may in some cases be filled with a wound packing material such as foam or gauze as described above. The applicator 180 of the fluidic connector 110 has a sealing surface that is placed over an aperture in the dressing 100 and is sealed to the top surface of the dressing 100. Either before, during, or after connection of the fluidic connector 110 to the dressing 100, the pump 150 is connected via the tube to the coupling 160, or is connected directly to the bridge 120. The pump is then activated, thereby supplying negative pressure to the wound. Application of negative pressure may be applied until a desired level of healing of the wound is achieved.

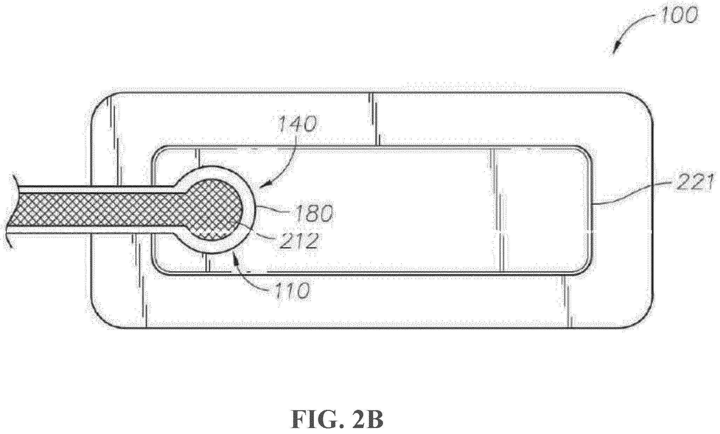

As shown in Figure 2B, the fluidic connector 110 preferably comprises an enlarged distal end, or head 140 that is in fluidic communication with the dressing 100 as will be described in further detail below. In one embodiment, the enlarged distal end has a round or circular shape. The head 140 is illustrated here as being positioned near an edge of the dressing 100, but may also be positioned at any location on the dressing. For example, some embodiments may provide for a centrally or off-centered location not on or near an edge or corner of the dressing 100. In some embodiments, the dressing 10 may comprise two or more fluidic connectors 110, each comprising one or more heads 140, in fluidic communication therewith. In a preferred embodiment, the head 140 may measure 30mm along its widest edge. The head 140 forms at least in part the applicator 180, described above, that is configured to seal against a top surface of the wound dressing.

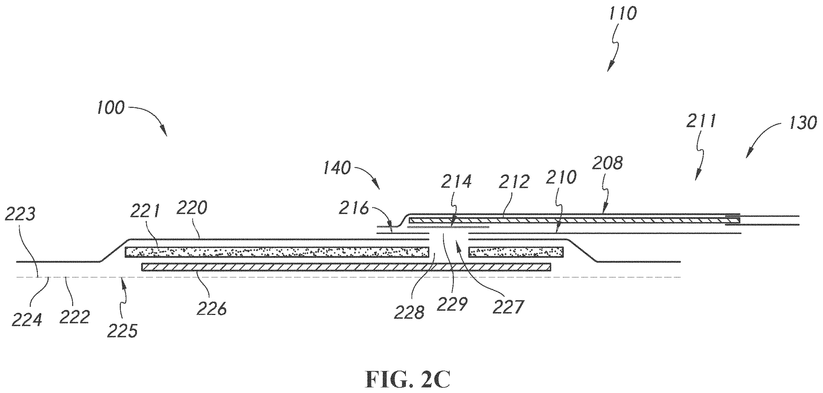

Figure 2C illustrates a cross-section through a wound dressing 100 similar to the wound dressing 10 as described in International Patent Publication WO2013175306 A2 along with fluidic connector 110. The wound dressing 100, which can alternatively be any wound dressing embodiment disclosed herein or any combination of features of any number of wound dressing embodiments disclosed herein, can be located over a wound site to be treated. The dressing 100 may be placed as to form a sealed cavity over the wound site. In a preferred embodiment, the dressing 100 comprises a top or cover layer, or backing layer 220 attached to an optional wound contact layer 222, both of which are described in greater detail below. These two layers 220, 222 are preferably joined or sealed together so as to define an interior space or chamber. This interior space or chamber may comprise additional structures that may be adapted to distribute or transmit negative pressure, store wound exudate and other fluids removed from the wound, and other functions which will be explained in greater detail below. Examples of such structures, described below, include a transmission layer 226 and an absorbent layer 221.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound.

As illustrated in Figure 2C, the wound contact layer 222 can be a polyurethane layer or polyethylene layer or other flexible layer which is perforated, for example via a hot pin process, laser ablation process, ultrasound process or in some other way or otherwise made permeable to liquid and gas. The wound contact layer 222 has a lower surface 224 and an upper surface 223. The perforations 225 preferably comprise through holes in the wound contact layer 222 which enable fluid to flow through the layer 222. The wound contact layer 222 helps prevent tissue ingrowth into the other material of the wound dressing. Preferably, the perforations are small enough to meet this requirement while still allowing fluid to flow therethrough. For example, perforations formed as slits or holes having a size ranging from 0.025 mm to 1.2 mm are considered small enough to help prevent tissue ingrowth into the wound dressing while allowing wound exudate to flow into the dressing. In some configurations, the wound contact layer 222 may help maintain the integrity of the entire dressing 100 while also creating an air tight seal around the absorbent pad in order to maintain negative pressure at the wound.

Some embodiments of the wound contact layer 222 may also act as a carrier for an optional lower and upper adhesive layer (not shown). For example, a lower pressure sensitive adhesive may be provided on the lower surface 224 of the wound dressing 100 whilst an upper pressure sensitive adhesive layer may be provided on the upper surface 223 of the wound contact layer. The pressure sensitive adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilized may be helpful to adhere the wound dressing 100 to the skin around a wound site. In some embodiments, the wound contact layer may comprise perforated polyurethane film. The lower surface of the film may be provided with a silicone pressure sensitive adhesive and the upper surface may be provided with an acrylic pressure sensitive adhesive, which may help the dressing maintain its integrity. In some embodiments, a polyurethane film layer may be provided with an adhesive layer on both its upper surface and lower surface, and all three layers may be perforated together.

A transmission layer 226 can be located above the wound contact layer 222. In some embodiments, the transmission layer can be a porous material. As used herein the transmission layer can be referred to as a spacer layer and the terms can be used interchangeably to refer to the same component described herein. This transmission layer 226 allows transmission of fluid including liquid and gas away from a wound site into upper layers of the wound dressing. In particular, the transmission layer 226 preferably ensures that an open-air channel can be maintained to communicate negative pressure over the wound area even when the absorbent layer has absorbed substantial amounts of exudates. The layer 226 should preferably remain open under the typical pressures that will be applied during negative pressure wound therapy as described above, so that the whole wound site sees an equalized negative pressure. The layer 226 may be formed of a material having a three-dimensional structure. For example, a knitted or woven spacer fabric (for example Baltex 7970 weft knitted polyester) or a non-woven fabric could be used. The three-dimensional material can comprise a 3D spacer fabric material similar to the material described in International Publication WO 2013/175306 A2 and International Publication WO2014/020440.

The wound dressing 100 incorporates or comprises one or more nitric oxide generating layers (e.g. a nitrite delivery layer, an acidic-group providing layer) as described herein this section or elsewhere in the specification. One of skill in the art will understand that the wound dressing 100 incorporates any of the one or more nitric oxide generating layers disclosed herein this section or elsewhere in the specification. One of skill in the art will also understand that the one or more nitric oxide generating layers may be incorporated as a whole component layer or a part of a component layer. In some embodiments, the one or more nitric oxide generating layers may be provided below the transmission layer 226. In some embodiments, the one or more nitric oxide generating layers may be provided above the wound contact layer 222. In certain embodiments, the one or more nitric oxide generating layers may replace the transmission layer 226, such that the one or more nitric oxide generating layers are provided between an absorbent layer 221 (described further below) and the wound contact layer 222. In some embodiments, the one or more nitric oxide generating layers can supplement or replace the absorbent layer 221. In some embodiments, the wound dressing 100 does not have the wound contact layer 222, and the one or more nitric oxide generating layers may be the lowermost layer of the wound dressing 100. The one or more nitric oxide generating layers may have same or substantially similar size and shape with the transmission layer 226 and/or the absorbent layer 221. In some embodiments, the one or more nitric oxide generating layers or components thereof (e.g., a nitrite providing layer as described herein) can be separate of the wound dressing 100. For example, the one or more nitric oxide generating layers or components thereof can be provided as a separate layer that can be placed on a wound, and the wound dressing 100 can be placed thereupon.

The one or more nitric oxide generating layers may be constructed to be flexible but stiff enough to withstand negative pressure, such that the one or more nitric oxide generating layers is not collapsed excessively and thereby may transmit negative pressure sufficiently to the wound when negative pressure is supplied to the wound dressing 100. The one or more nitric oxide generating layers may be constructed to include sufficient number or size of pores to enable transmission of negative pressure. The one or more nitric oxide generating layer may include an aperture or hole, for example, under the port, to transmit negative pressure and/or wound fluid. Further, the one or more nitric oxide generating layers may have suitable thickness(es) to transmit suitable negative pressure to the wound. For example, the one or more nitric oxide generating layers may have a thickness of about 1 mm to 10 mm, or 1 mm to 7 mm, or 1.5 mm to 7 mm, or 1.5 mm to 4 mm, or 2 mm to 3 mm. In some embodiments, the one or more nitric oxide generating layers may have a thickness of approximately 2 mm.

In some embodiments, the layer 221 of absorbent material is provided above the transmission layer 226. The absorbent material, which can comprise a foam or non-woven natural or synthetic material, and which may optionally comprise a super-absorbent material, forms a reservoir for fluid, particularly liquid, removed from the wound site. In some embodiments, the layer 221 may also aid in drawing fluids towards the backing layer 220.

The material of the absorbent layer 221 may also prevent liquid collected in the wound dressing 100 from flowing freely within the dressing, and preferably acts so as to contain any liquid collected within the dressing. The absorbent layer 221 also helps distribute fluid throughout the layer via a wicking action so that fluid is drawn from the wound site and stored throughout the absorbent layer. This helps prevent agglomeration in areas of the absorbent layer. The capacity of the absorbent material must be sufficient to manage the exudates flow rate of a wound when negative pressure is applied. Since in use the absorbent layer experiences negative pressures the material of the absorbent layer is chosen to absorb liquid under such circumstances. A number of materials exist that are able to absorb liquid when under negative pressure, for example superabsorber material. The absorbent layer 221 may typically be manufactured from ALLEVYN^{™} foam, Freudenberg 114-224-4 or Chem-Posite^{™}11C-450. In some embodiments, the absorbent layer 221 may comprise a composite comprising superabsorbent powder, fibrous material such as cellulose, and bonding fibers. In a preferred embodiment, the composite is an air-laid, thermally-bonded composite.

In some embodiments, the absorbent layer 221 is a layer of non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers leads to strong capillary action in the fibrous pad which helps distribute liquid. In this way, the super-absorbent material is efficiently supplied with liquid. The wicking action also assists in bringing liquid into contact with the upper cover layer to aid increase transpiration rates of the dressing.

An aperture, hole, or orifice 227 is preferably provided in the backing layer 220 to allow a negative pressure to be applied to the dressing 100. The fluidic connector 110 is preferably attached or sealed to the top of the backing layer 220 over the orifice 227 made into the dressing 100, and communicates negative pressure through the orifice 227. A length of tubing may be coupled at a first end to the fluidic connector 110 and at a second end to a pump unit (not shown) to allow fluids to be pumped out of the dressing. Where the fluidic connector is adhered to the top layer of the wound dressing, a length of tubing may be coupled at a first end of the fluidic connector such that the tubing, or conduit, extends away from the fluidic connector parallel or substantially to the top surface of the dressing. The fluidic connector 110 may be adhered and sealed to the backing layer 220 using an adhesive such as an acrylic, cyanoacrylate, epoxy, UV curable or hot melt adhesive. The fluidic connector 110 may be formed from a soft polymer, for example a polyethylene, a polyvinyl chloride, a silicone or polyurethane having a hardness of 30 to 90 on the Shore A scale. In some embodiments, the fluidic connector 110 may be made from a soft or conformable material.

Optionally, the absorbent layer 221 includes at least one through hole 228 located so as to underlie the fluidic connector 110. The through hole 228 may in some embodiments be the same size as the opening 227 in the backing layer, or may be bigger or smaller. As illustrated in Figure 2C a single through hole can be used to produce an opening underlying the fluidic connector 110. It will be appreciated that multiple openings could alternatively be utilized. Additionally, should more than one port be utilized according to certain embodiments of the present disclosure one or multiple openings may be made in the absorbent layer in registration with each respective fluidic connector. Although not essential to certain embodiments of the present disclosure the use of through holes in the super-absorbent layer may provide a fluid flow pathway which remains unblocked in particular when the absorbent layer is near saturation.

The aperture or through-hole 228 is preferably provided in the absorbent layer 221 beneath the orifice 227 such that the orifice is connected directly to the transmission layer 226 as illustrated in Figure 2C. This allows the negative pressure applied to the fluidic connector 110 to be communicated to the transmission layer 226 without passing through the absorbent layer 221. This ensures that the negative pressure applied to the wound site is not inhibited by the absorbent layer as it absorbs wound exudates. In other embodiments, no aperture may be provided in the absorbent layer 221, or alternatively a plurality of apertures underlying the orifice 227 may be provided. In further alternative embodiments, additional layers such as another transmission layer or an obscuring layer such as described with in International Patent Publication WO2014/020440, may be provided over the absorbent layer 221 and beneath the backing layer 220.

The backing layer 220 is preferably gas impermeable, but moisture vapor permeable, and can extend across the width of the wound dressing 100. The backing layer 220, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way, an effective chamber is made between the backing layer 220 and a wound site where a negative pressure can be established. The backing layer 220 is preferably sealed to the wound contact layer 222 in a border region around the circumference of the dressing, ensuring that no air is drawn in through the border area, for example via adhesive or welding techniques. The backing layer 220 protects the wound from external bacterial contamination (bacterial barrier) and allows liquid from wound exudates to be transferred through the layer and evaporated from the film outer surface. The backing layer 220 preferably comprises two layers; a polyurethane film and an adhesive pattern spread onto the film. The polyurethane film is preferably moisture vapor permeable and may be manufactured from a material that has an increased water transmission rate when wet. In some embodiments, the moisture vapor permeability of the backing layer increases when the backing layer becomes wet. The moisture vapor permeability of the wet backing layer may be up to about ten times more than the moisture vapor permeability of the dry backing layer.

The absorbent layer 221 may be of a greater area than the transmission layer 226, such that the absorbent layer overlaps the edges of the transmission layer 226, thereby ensuring that the transmission layer does not contact the backing layer 220. This provides an outer channel of the absorbent layer 221 that is in direct contact with the wound contact layer 222, which aids more rapid absorption of exudates to the absorbent layer. Furthermore, this outer channel ensures that no liquid is able to pool around the circumference of the wound cavity, which may otherwise seep through the seal around the perimeter of the dressing leading to the formation of leaks. As illustrated in Figure 2C, the absorbent layer 221 may define a smaller perimeter than that of the backing layer 220, such that a boundary or border region is defined between the edge of the absorbent layer 221 and the edge of the backing layer 220.

As shown in Figure 2C, one embodiment of the wound dressing 100 comprises an aperture 228 in the absorbent layer 221 situated underneath the fluidic connector 110. In use, for example when negative pressure is applied to the dressing 100, a wound facing portion of the fluidic connector may thus come into contact with the transmission layer 226, which can thus aid in transmitting negative pressure to the wound site even when the absorbent layer 221 is filled with wound fluids. Some embodiments may have the backing layer 220 be at least partly adhered to the transmission layer 226. In some embodiments, the aperture 228 is at least 1-2 mm larger than the diameter of the wound facing portion of the fluidic connector 11, or the orifice 227.

In particular for embodiments with a single fluidic connector 110 and through hole, it may be preferable for the fluidic connector 110 and through hole to be located in an off-center position as illustrated in Figure 2B. Such a location may permit the dressing 100 to be positioned onto a patient such that the fluidic connector 110 is raised in relation to the remainder of the dressing 100. So positioned, the fluidic connector 110 and the filter 214 may be less likely to come into contact with wound fluids that could prematurely occlude the filter 214 so as to impair the transmission of negative pressure to the wound site.

Similar to the embodiments of wound dressings described above, some wound dressings comprise a perforated wound contact layer with silicone adhesive on the skin-contact face and acrylic adhesive on the reverse. In some embodiments, the wound contact layer may be constructed from polyurethane, polyethylene or polyester. In some embodiments, the wound contact layer may comprise an absorbent material. Above this bordered layer sits a transmission layer. Above the transmission layer, sits an absorbent layer. The absorbent layer can include a superabsorbent non-woven (NW) pad. The absorbent layer can over-border the transmission layer by approximately 5mm at the perimeter. The absorbent layer can have an aperture or through-hole toward one end. The aperture can be about 10 mm in diameter. Over the transmission layer and absorbent layer lies a backing layer. The backing layer can be a high moisture vapor transmission rate (MVTR) film, pattern coated with acrylic adhesive. The high MVTR film and wound contact layer encapsulate the transmission layer and absorbent layer, creating a perimeter border of approximately 20 mm. The backing layer can have a 10 mm aperture that overlies the aperture in the absorbent layer. Above the hole can be bonded a fluidic connector that comprises a liquid-impermeable, gas-permeable semi-permeable membrane (SPM) or filter that overlies the aforementioned apertures.

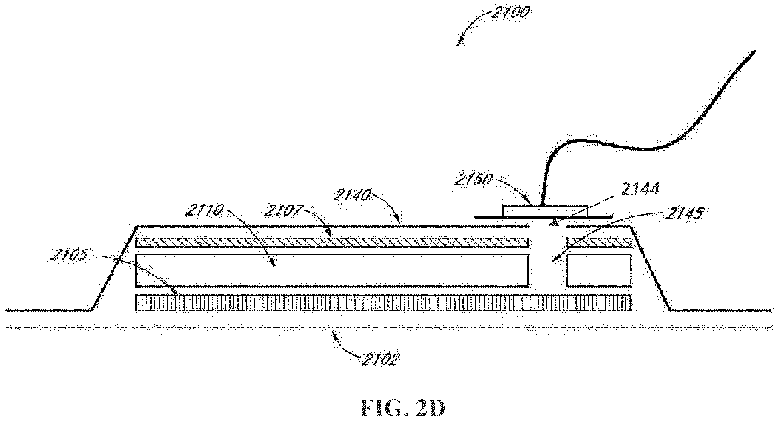

Figure 2D depicts an embodiment of a wound dressing, similar to the wound dressings of Figures 2A-2C. With reference to Figure 2D, a masking or obscuring layer 2107 can be positioned beneath at least a portion of the backing layer 2140. In some embodiments, the obscuring layer 2107 can have any of the same features, materials, or other details of any of the other embodiments of the obscuring layers disclosed herein, including but not limited to having any viewing windows or holes. Examples of wound dressings with obscuring layers and viewing windows are described in International Patent Publication WO2014/020440. Additionally, the obscuring layer 2107 can be positioned adjacent to the backing layer, or can be positioned adjacent to any other dressing layer desired. In some embodiments, the obscuring layer 2107 can be adhered to or integrally formed with the backing layer. Preferably, the obscuring layer 2107 is configured to have approximately the same size and shape as the absorbent layer 2110 so as to overlay it. As such, in these embodiments the obscuring layer 2107 will be of a smaller area than the backing layer 2140.

Preferably the absorbent layer 2110 and the obscuring layer 2107 include at least one through hole 2145 located so as to underlie the port 2150. Of course, the respective holes through these various layers 2107, 2140, and 2110 may be of different sizes with respect to each other. As illustrated in Figure 2D a single through hole can be used to produce an opening underlying the port 2150. In certain embodiments, the port may be replaced with or used in combination with a fluidic connector such as depicted in Figure 2C. It will be appreciated that multiple openings could alternatively be utilized. Additionally, should more than one port be utilized according to certain embodiments of the present disclosure one or multiple openings may be made in the absorbent layer and the obscuring layer in registration with each respective port. Although not essential to certain embodiments of the present disclosure the use of through holes in the super-absorbent layer may provide a fluid flow pathway which remains unblocked in particular when the absorbent layer 2110 is near saturation.

The aperture or through-hole 2144 may be provided in the absorbent layer 2110 and the obscuring layer 2107 beneath the orifice 2144 such that the orifice is connected directly to the transmission layer 2105. This allows the negative pressure applied to the port 2150 to be communicated to the transmission layer 2105 without passing through the absorbent layer 2110. This ensures that the negative pressure applied to the wound site is not inhibited by the absorbent layer as it absorbs wound exudates. In other embodiments, no aperture may be provided in the absorbent layer 2110 and/or the obscuring layer 2107, or alternatively a plurality of apertures underlying the orifice 2144 may be provided.

In some embodiments, the obscuring layer 1404 can help to reduce the unsightly appearance of a dressing during use, by using materials that impart partial obscuring or masking of the dressing surface. The obscuring layer 1404 in one embodiment only partially obscures the dressing, to allow clinicians to access the information they require by observing the spread of exudate across the dressing surface. The partial masking nature of this embodiment of the obscuring layer enables a skilled clinician to perceive a different color caused by exudate, blood, by-products etc. in the dressing allowing for a visual assessment and monitoring of the extent of spread across the dressing. However, since the change in color of the dressing from its clean state to a state containing exudate is only a slight change, the patient is unlikely to notice any aesthetic difference. Reducing or eliminating a visual indicator of wound exudate from a patient's wound is likely to have a positive effect on their health, reducing stress for example.

In some embodiments, the obscuring layer can be formed from a non-woven fabric (for example, polypropylene), and may be thermally bonded using a diamond pattern with 19% bond area. In various embodiments, the obscuring layer can be hydrophobic or hydrophilic. Depending on the application, in some embodiments, a hydrophilic obscuring layer may provide added moisture vapor permeability. In some embodiments, however, hydrophobic obscuring layers may still provide sufficient moisture vapor permeability (i.e., through appropriate material selection, thickness of the obscuring layer), while also permitting better retention of dye or color in the obscuring layer. As such, dye or color may be trapped beneath the obscuring layer. In some embodiments, this may permit the obscuring layer to be colored in lighter colors or in white. In the preferred embodiment, the obscuring layer is hydrophobic. In some embodiments, the obscuring layer material can be sterilizable using ethylene oxide. Other embodiments may be sterilized using gamma irradiation, an electron beam, steam or other alternative sterilization methods. Additionally, in various embodiments the obscuring layer can colored or pigmented, e.g., in medical blue. The obscuring layer may also be constructed from multiple layers, including a colored layer laminated or fused to a stronger uncolored layer. Preferably, the obscuring layer is odorless and exhibits minimal shedding of fibers.

### Multi-Layered Dressing for Use Without Negative Pressure

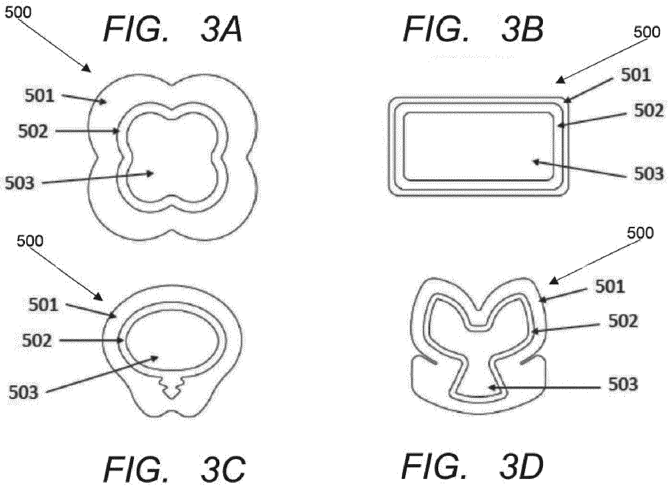

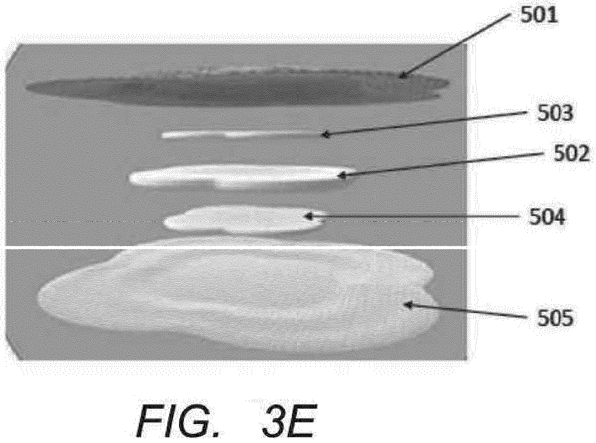

Figures 3A-3D illustrates various embodiments of a wound dressing 500 that can be used for healing a wound without negative pressure. Figure 3E illustrates a cross-section of the wound dressing in Figures 3A-3D. As shown in the dressings of Figures 3A-3E, the wound dressings can have multiple layers similar to the dressings described with reference to Figures 2A-2D except the dressings of Figures 3A-E do not include a port or fluidic connector. The wound dressings of Figures 3A-E can include a cover layer 501 and an optional wound contact layer 505 as described herein. In some embodiments, the cover layer 501 may be permeable to moisture and/or air. The wound dressing can include various layers positioned between the wound contact layer 505 and cover layer 501. For example, the dressing can include one or more absorbent layers or one or more transmission layers as described herein with reference to Figures 2A-2C.

As shown in Figures 3A-3E, the dressing 500 may include a perforated wound contact layer 505 and a top film 501. Further components of the wound dressing 500 include a foam layer 504, such as a layer of polyurethane hydrocellular foam, of a suitable size to cover the recommended dimension of wounds corresponding to the particular dressing size chosen. An optional layer of activated charcoal cloth (not shown) of similar or slightly smaller dimensions than layer 504 may be provided to allow for odour control. An absorbent layer 502, such as a layer of superabsorbent air-laid material containing cellulose fibres and a superabsorbent polyacrylate particulates, is provided over layer 504, of dimensions slightly larger than layer 504, and allows for an overlap of superabsorbent material and acts as leak prevention. A masking or obscuring layer 503, such as a layer of three-dimensional knitted spacer fabric, is provided over layer 502, providing protection from pressure, while allowing partial masking of the top surface of the superabsorber where coloured exudate would remain. In this embodiment this is of smaller dimension (in plan view) than the layer 502, to allow for visibility of the edge of the absorbent layer, which can be used by clinicians to assess whether the dressing needs to be changed.

The wound dressing 500 incorporates or comprises one or more nitric oxide generating layers (e.g. a nitrite delivery layer, an acidic-group providing layer) as described herein this section or elsewhere. One of skill in the art will understand that the wound dressing 500 incorporates any of the one or more nitric oxide generating layers disclosed herein this section or elsewhere in the specification. One of skill in the art will also understand that the one or more nitric oxide generating layers may be incorporated as a whole component layer or a part of a component layer. The nitric oxide generating layer(s) is provided below the cover layer 501. In some embodiments, the one or more nitric oxide generating layers may be provided above the wound contact layer 505. In certain embodiments, the dressing 500 may not include the wound contact layer 505, such that one of the nitric oxide generating layers may be the lowermost layer and be configured to touch the wound surface. In some embodiments, the one or more nitric oxide generating layers may be provided below the foam layer 504. In embodiments, the nitric oxide generating layers may replace the foam layer 504. In some embodiments, the dressing 500 may include only the cover layer 501 and the one or more nitric oxide generating layers. In some embodiments, the one or more nitric oxide generating layers or components thereof (e.g., a nitrite providing layer as described herein) can be separate of the wound dressing 500. For example, the one or more nitric oxide generating layers or components thereof can be provided as a separate layer that can be placed on a wound, and the wound dressing 500 can be placed thereupon.

As described previously herein, the one or more nitric oxide generating layers, may be incorporated into or used with commercially available dressings, such as ALLEVYN^{™} foam, ALLEVYN^{™} Life, ALLEVYN^{™} Adhesive, ALLEVYN^{™} Gentle Border, ALLEVYN^{™} Gentle, ALLEVYN^{™} Ag Gentle Border, ALLEVYN^{™} Ag Gentle, Opsite Post-Op Visible. In some embodiments, the wound dressing 500 may include the cover layer 501, the wound contact layer 505 and the nitric oxide generating layers sandwiched therebetween. In some embodiments, the wound dressing 500 may include the cover layer 501, the absorbent layer 502, the nitric oxide generating layers below the absorbent layer 502, and the wound contact layer 505.

Further details regarding wound dressings that may be combined with or be used in addition to the embodiments described herein, are found in U.S. Patent No. 9,877,872, issued on January 30, 2018, titled "WOUND DRESSING AND METHOD OF TREATMENT," including further details relating to embodiments of wound dressings, the wound dressing components and principles, and the materials used for the wound dressings.

### Multilayered Wound Dressing with an Integrated Source of Negative Pressure

In some embodiments, a source of negative pressure (such as a pump) and some or all other components of the TNP system, such as power source(s), sensor(s), connector(s), user interface component(s) (such as button(s), switch(es), speaker(s), screen(s), etc.) and the like, can be integral with the wound dressing, such as the dressings described above in relation to Figures 1-3D. Additionally, some embodiments related to wound treatment comprising a wound dressing described herein may also be used in combination or in addition to those described in International Application WO 2016/174048 and International Patent Application PCT/EP2017/055225, filed on March 6, 2017, entitled "WOUND TREATMENT APPARATUSES AND METHODS WITH NEGATIVE PRESSURE SOURCE INTEGRATED INTO THE WOUND DRESSING," including further details relating to embodiments of wound dressings, the wound dressing components and principles, and the materials used for the wound dressings and wound dressing components.

In some embodiments, the pump and/or other electronic components can be configured to be positioned adjacent to or next to the absorbent and/or transmission layers in the wound dressing so that the pump and/or other electronic components are still part of a single apparatus to be applied to a patient with the pump and/or other electronics positioned away from the wound site.

### Nitric Oxide Generating Layers

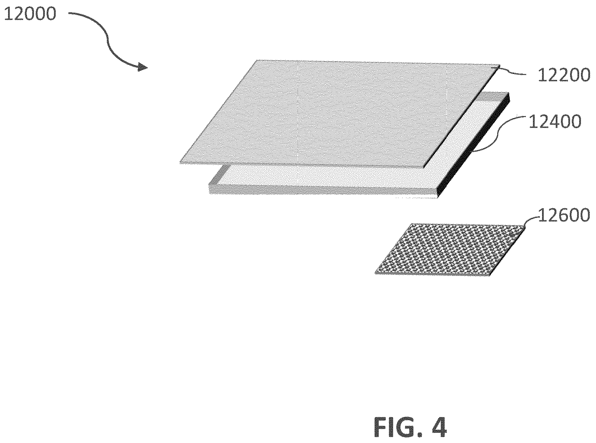

FIGS. 4-5 illustrate a wound dressing 12000 including nitric oxide generating layers according to some embodiments. In the illustrated embodiments, the wound dressing 12000 may include a cover layer 12200, an activator layer 12400, and a nitric oxide source layer 12600. In some embodiments, the wound dressing 12000 may include additional layers, as further described herein. One of skill in the art will understand that although the various sections of the dressing may be referred to as "layers," such sections may be in other suitable shapes or configurations.

The cover layer 12200 may be gas impermeable, but moisture vapor permeable, and can extend across the width of the wound dressing 12000. The cover layer 12200, which may for example be a polyurethane film (for example, Elastollan SP9109 or Elastollan SP806) having a pressure sensitive adhesive on one side, may be impermeable to gas and this layer may thus operate to cover the wound and to seal a wound cavity over which the wound dressing is placed. Therefore, a chamber or a sealed wound space is made between the cover layer 12200 and the wound site. In some embodiments, negative pressure can be established within the chamber or the sealed wound space made between the cover layer 12200 and the wound site. The cover layer 12200 protects the wound from external bacterial contamination (bacterial barrier) and allows liquid from wound exudates to be transferred through the layer and evaporated from the film outer surface. The cover layer 12200 may include two or more layers, for example, a polyurethane film and an adhesive pattern spread onto the film. In certain examples, the polyurethane film may be moisture vapor permeable and may be manufactured from a material that has an increased water transmission rate when wet. In some embodiments, the moisture vapor permeability of the cover layer increases when the cover layer becomes wet. The moisture vapor permeability of the wet cover layer may be up to about ten times more than the moisture vapor permeability of the dry cover layer. In some embodiments, the cover layer 12200 may be replaced or supplemented with an additional wound dressings described elsewhere herein, such that the additional wound dressings are positioned above the nitric oxide generating layers. The cover layer may also be shower proof, such that a dressing incorporating such a cover layer may be used in the shower. The cover layer may be configured such that nitric oxide does not immediately escape through the cover layer, meaning that the cover layer is nitric oxide impermeable or semi-impermeable, thereby trapping nitric oxide against the tissue such that nitric oxide can interact with the body of a user. One of skill in the art will understand that the cover layer may be made to be both vapor permeable, but nitric oxide impermeable.

The nitric oxide source layer 12600 may provide one or more nitric oxide-releasing agents at the wound site. The nitric oxide-releasing agent can include any chemical entity that yields nitric oxide at the wound site when activated or otherwise stimulated to do so. In some embodiments, the nitric oxide-releasing agent can include nitrite ion, a nitrite salt, organic and inorganic nitrites, or any pharmacologically acceptable source of nitrite such that the nitrite ion can be reduced to produce nitric oxide at the wound site. For example, the nitric oxide source layer 12600 and/or element may include one or more of ammonium nitrite, lithium nitrite, calcium nitrite, sodium nitrite, potassium nitrite. In some embodiments, the nitric oxide source layer may be a suitable material layer or element that includes alkali metal nitrites and/or alkaline earth metal nitrites. In certain embodiments, the nitrites may include: LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, Ra(NO₂)₂ or any other suitable nitrite. In some embodiments, a precursor of nitrite ions, such as nitrous acid, nitrate ions, nitroprusside ions, or any pharmacologically acceptable salts thereof may be used as the source of the nitrite. In some embodiments, the nitric oxide-releasing agents may include nitrites such as nitro-functionalized compounds. For example, the nitric oxide-releasing agents may include nitroglycerine, isoamyl nitrite, isorbide mononitrate, N-(Ethoxycarbonyl)-3-(4-morpholinyl)sydnoneimine; 3-morpholinosydnonimine; 1,2,3,4-Oxatriazolium; 5-amino-3-(3,4-di-chlorophenyl)-chloride; 1,2,3,4-Oxatriazolium; 5-amino-3-(chloro-2-methyl-phenyl)chloride; 1,2,3,4-Oxatriazolium, 3-(3-chloro-2-methylphenyl)-5-[[[cyanomethylamino]carbonyl]amino]-hydroxide inner salt; S-nitroso-N-acetyl-(D,L)-penicillamine; l-[(4',5'-Bis(carboxymethoxy)-2l-nitrophenyl)methoxy]-2-oxo-3,3,diethyl-l-triazene dipotassium salt; and [l-(4', 5'-Bis(carboymethoxy)-2'-nitropheyl)methoxy]-2-oxo-3,3-diethyl-1-triazine diacetoxymethyl ester.

In some embodiments, the nitric oxide-releasing agent of the nitric oxide source layer 12600 can include diazeniumdiolates, including O-alkylated diazeniumdiolate, O-derivatized diazeniumdiolate, and non-O-derivatized diaziniumdiolate. For example, the nitric oxide-releasing agent can include diethylamine/NO, V-PYRRO/NO and/or Spermine/NO. In some embodiments, the nitric oxide-releasing agent of the nitric oxide source layer 12600 can include S-nitrosothiols, such as S-nitro-gluthathione, S-nitroso-N-acetylcystein, S-nitroso-acetylpenicillamine. In some embodiments, the nitric oxide-releasing agent of the nitric oxide source layer 12600 may include silica, or silica nano-particles modified with nitric oxide. In some embodiments, the nitric oxide-releasing agent can be a polymer modified with nitric oxide to include nitric oxide. For example, polyethyleneimine, polypropyleneimines, polybutyleneimines, polyurethanes or polyamides can be modified with nitric oxide to form diazeniumdiolate. In some embodiments, the nitric oxide source layer 12600 may be constructed from such polymers modified with nitric oxide. Further examples of the nitric oxide-releasing agents are provided in International Publication No. WO 2006/058318, and Liang et al., "Nitric oxide generating/releasing materials", Future Science OA, 1 (1) (2015).

In some embodiments, the nitric oxide source layer 12600 may include a nitric oxide-releasing agent (e.g. sodium nitrite) in an aqueous solution. For example, the nitric oxide source layer 12600 may include a material imbibed with the nitric oxide-releasing agent (e.g. sodium nitrite) solution. In some embodiments, the nitric oxide source layer 12600 may include a dry nitric oxide-releasing agent (e.g. sodium nitrite) in solid form.

The nitric oxide source layer 12600 may include a mesh, a foam, a gel or any other material suitable for containing the nitric oxide-releasing agent. For example, the nitric oxide source layer 12600 may include a mesh imbibed with the nitric oxide-releasing agent (e.g. sodium nitrite) solution. The mesh may be knitted, woven or non-woven. The mesh may be made of a polymeric material, for example, viscose, polyamide, polyester, polypropylene or a combination thereof. In some embodiments, the nitric oxide source layer 12600 may include polypropylene, polyester, polyurethane, polyvinyl chloride, polyamide, viscose, polyester, polypropylene and/or cellulose. As described herein, the nitric oxide source layer 12600 may be constructed from one or more polymers modified with nitric oxide. The nitric oxide source layer 12600 could also be made of a hydrogel without acidic groups to prevent reaction with nitrite ions to emit nitric oxide. In some embodiments, the nitric oxide source layer 12600 may be constructed from a colored material, such that the nitric oxide source layer 12600 can be visible to assist positioning of the wound dressing 12000 during application to the wound, and to reduce the risk of incomplete removal of the nitric oxide source layer 12600 from the wound after treatment. The nitric oxide source layer 12600 may be fully or semi-permeable to the diffusion of nitric oxide.

In some embodiments, the nitric oxide source layer 12600 is the lowermost layer of the dressing 12000, such that the nitric oxide source layer 12600 may contact the wound. In some embodiments, the nitric oxide source layer 12600 may be positioned within and/or over the wound. The nitric oxide source layer may be constructed such that the nitric oxide source layer 12600 do not substantially adhere to the skin or wound, or cause da mage to the wound when in contact with the wound. In some embodiments, the dressing 12000 may include one or more layers, for example a wound contact layer, beneath the nitric oxide source layer 12600. In some embodiments, the wound contact layer may comprise an absorbent material. In some embodiments, the wound dressing 12000 may include two or more nitric oxide source layers. For example, the wound dressing 12000 may include 2, 3, 4, 5, 6, 7 or more nitric oxide source layers. In some embodiments, the nitrite oxide source layer 12600 can be separate of the wound dressing 12000. For example, the nitrite oxide source layer 12600 can be provided as a separate layer that can be placed on a wound, and the wound dressing 12000 can be placed thereupon. The nitric oxide-releasing agent may be incorporated into the nitrite providing layer to provide a nitrite dose (e.g., a sodium nitrite dose), in M (Molar) of about: 0.01 to 5.0, 0.5 to 4.5, 1.0 to 3.0, 1.0 to 2.0, and/or 1.0 to 1.5. For example, the dose may be about 0.50 M, about 0.01 M, about 1.5 M, about 2 M, or about 2.5 M.

The activator layer 12400 may contain chemical agents, functional groups or moieties which can activate and/or facilitate release of nitric oxide from the nitric oxide-releasing agent. For example, protons or acidic environment promotes the reduction of nitrites to nitric oxide, and the activator layer 12400 may include acidic groups or moieties which may provide protons in aqueous environment, thereby lowering the pH at the site of application. In certain embodiments, the acidic groups or moieties are immobilized at the activator layer 12400, for example on the surface of the activator layer 12400. The acidic groups or moieties may be covalently bonded at the activator layer 12400. In some embodiments, the activator layer 12400 may include an acidic solution. The activator layer 12400 may include a mesh, a foam, a gel or any other material suitable for containing acid groups or moieties. In embodiments, the activator layer 12400 is positioned above the nitric oxide source layer 12600 or the activator layer 12400 may be positioned below the nitric oxide source layer 12600. In some embodiments, the activator layer 12400 may include proton sources such as water, methanol, ethanol, propanols, butanols, pentanols, hexanols, phenols, naphtols or polyols; aqueous acidic buffers such as phosphates, succinates, carbonates, acetates, formats, propionates, butyrates, fatty acids, amino acids, or ascorbic acids; or any suitable enzymatic or catalytic compounds. In some embodiments, body fluid such as blood, lymph, bile, or wound exudate may function as the activator, and can assist the activator layer 12400. In some embodiments, the wound dressing 12000 may not include the activator layer 12400, and wound fluid or wound exudate may function as the activator. Further examples of the activators for the nitric oxide-releasing agents are provided in International Publication No. WO 2006/058318, and Liang et al., "Nitric oxide generating/releasing materials", Future Science OA, 1 (1) (2015).

In some embodiments, the wound dressing 12000 may include two or more nitric oxide source layers and/or two or more activator layers. For example, the wound dressing 12000 may include 2, 3, 4, 5, 6, 7 or more nitric oxide source layers and/or activator layers.

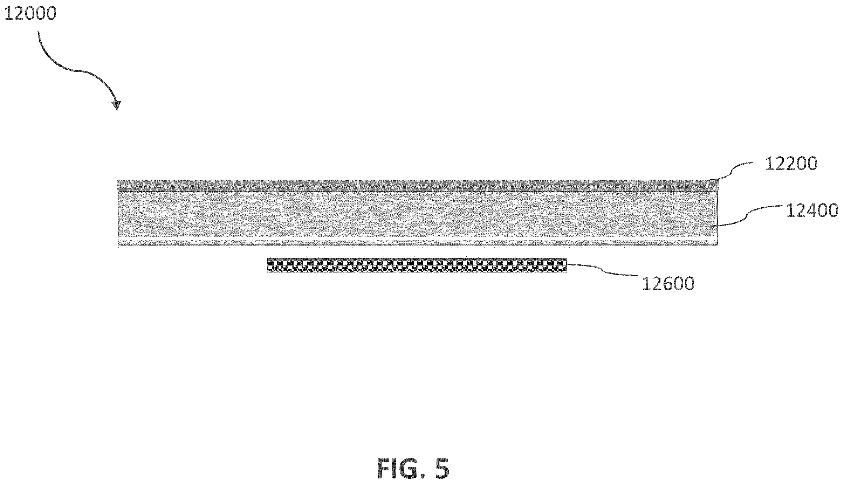

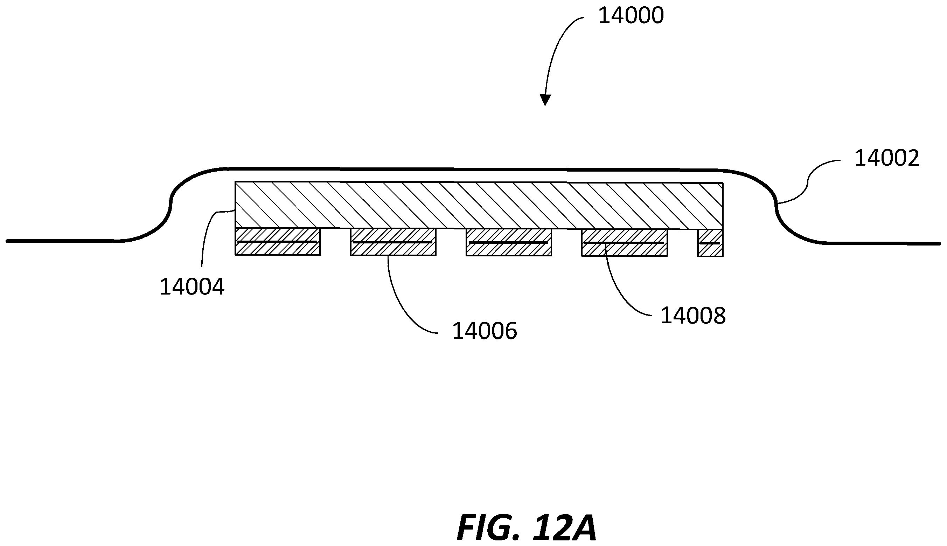

In some embodiments, the activator layer 12400 includes hydrogel, such that the activator layer 12400 can absorb the wound exudate. In certain examples, the activator layer 12400 may be constructed of a xerogel. The activator layer 12400 may be constructed from any suitable materials disclosed herein. The gel of the activator layer 12400 may be presented in different physical formats. For example, the activator layer 12400 may be foamed during curing. The hydrogel may be poured into a foam and then cured in the foam. In some embodiments, the activator layer 12400 may be perforated through its thickness. The perforations may be sized to allow fluid absorption and for the desired therapeutic dose of nitric oxide to be released from the wound dressing. For example, the perforations may have a diameter sized approximately between 0.1 mm and 10 mm, between 0.15 mm and 7 mm, between 0.2 mm and 5 mm, between 0.5 mm and 4 mm or between 0.7 mm and 3 mm. The perforations may have a circular shape, a square shape, a triangular shape, or any other suitable shape. The foamed construction and/or the perforations may contribute to fluid handling capabilities of the activator layer. In some embodiments, the activator layer 12400 illustrated in Figures 4 and 5 may comprise an absorbent layer 14004 and gel material or gel layer 14006 as described with reference to Figures 12A-I below.

In some embodiments, an activator material for the activator layer may be provided as a dispensable composition, for example as a prepolymer solution or otherwise malleable form, instead of being provided as the activator layer such as the activator layer 12400, such that it can be applied over the wound and/or around the wound more freely. For example, the activator material may be provided as gel prepolymer solution, such that it can be applied closely to or around a wound having an irregular shape size by a clinician. In some embodiments, the activator material, such as the gel prepolymer solution, may be provided in and/or applied with a syringe, and the gel prepolymer solution may have a viscosity suitable to be dispensed from the syringe. The activator material can be also formulated such that it can be rapidly cured and no longer flows once applied to or around the wound. The activator material may include an evaporative solvent, such as isopropanol. The activator material can have a suitable secondary curing mechanism, such as photoinitiated acrylate functionality. In some embodiments, the activator material can be provided as a reactive two-part system. For example, a first part and a second part may be provided to be mixed to result in polymer formation immediately before dispensing. In some embodiments, the first part and the second part may be oppositely charged flowable gels, such that they can interact on mixing to provide gels that do not flow substantially. In some embodiments, the activator material may include a material such as a gel which change in response to the change in environment. For example, the activator material may include a material such as certain pluronics, such that it can be cured once the temperature changes as being applied from the dispenser or syringe to the skin. The activator material may be applied such that it can interact with nitrite from the nitric oxide source layer 12600 (which may provide nitrite) to generate nitric oxide. Once the activator material is applied and cured or does not flow otherwise, the cover layer 12200 may be applied.

Once the dressing 12000 is activated, for example by placing the activator layer 12400 in contact with the nitric oxide source layer 12600, nitric oxide-releasing agents from the nitric oxide source layers 12600 releases nitric oxide. For example, in some embodiments, nitrites can be reduced to nitric oxide in the presence of an acidic environment provided by the activator layer 12400 as shown below:

NO₂⁻ + H⁺ ⇄ HNO₂ (1)

2HNO₂ ⇄ H₂O + N₂O₃ (2)

N₂O₃ ⇄ NO + NO₂ (3)

The activator layer 12400 and the nitric oxide source layer 12600 may be positioned such that the nitric oxide-releasing agents can react to provide nitric oxide. For example, the activator layer 12400 and the nitric oxide source layer 12600 may be in contact with each other within the dressing 12000 when in use. In some embodiments, one or more additional layers may be positioned between the activator layer 12400 and the nitric oxide source layer 12600. In some embodiments, the activator layer 12400 and the nitric oxide source layer 12600 may be fluidically isolated from each other before applying the dressing 12000 to the patient to prevent premature release of nitric oxide. For example, the nitric oxide source layer 12600 may be provided in a packaging separate from the rest of the dressing 12000. Once the dressing 12000 is activated, the nitric oxide-releasing agents from the nitric oxide source layer 12600 may disperse within the dressing 12000. In some embodiments, the nitric oxide-releasing agents may be dissolved in wound exudate and wound exudate may facilitate dispersal of the nitric oxide-releasing agents. At least a portion of the nitric oxide-releasing agents would react to release nitric oxide in the presence of the activators of the activator layer 12400. The generated nitric oxide may diffuse into the wound or be delivered to the wound by any suitable mechanisms. In some embodiments, the generated nitric oxide may not be delivered immediately or at all and is instead held within the dressing, for example by a selectively permeable membrane, such that the nitric oxide may prevent growth of or kill microbes within the dressing.

In some embodiments, the wound dressing 12000 can include a reducing agent to facilitate reduction of the nitric oxide-releasing agent (e.g. nitrite ion) to nitric oxide. Physiologically acceptable examples of such reducing agents include but are not limited to: iodide anion, ascorbic acid, ascorbate (e.g. sodium ascorbate), isoascorbates (e.g. sodium isoascorbate), hydroquinone, butylated quinone, tocopherol, butylated hydroquinone, hydroquinone varients, butylated hydroxyanisole, butylated hydroxytoluene, beta-carotene, potassium iodide, ascorbate variants, iso-ascorbate variants, any other suitable reducing agent, and/or any of the antioxidants and/or reducing agents described herein. The reducing agent may be included in one or more layers of the wound dressing 12000. For example, the reducing agent may be included in the cover layer 12200, the activator layer 12400, the nitric oxide source layer 12600, the wound contact layer (e.g., 222, 505), and/or any suitable layers of nitric oxide generating wound dressings described herein. The reducing agent may be incorporated to the one or more layers, for example, by physical entrapment, physical blending, coating, covalent bonding, or any other suitable methods. The reducing agent may be incorporated into the dressing and into the appropriate layer, such as a hydrogel activating layer, at a w/w % of about: 0.01 to 5.0%, 0.1 to 4.5%, 1.0 to 3.0%, 1.0 to 1.5%, and/or 1.5 to 2.5%. For example, the w/w% may be about 0.02%, about 0.03%, about 0.8%, about 1.2%, about 1.4%, or about 2.43%. Higher levels of reducing agent may lead to increased production of nitric oxide; however, very high levels of reducing agent may become toxic.

As described herein, the nitric oxide source layer may include nitrite and may be referred to as a nitrite delivery layer or a nitrite providing layer in this specification. As described herein, the activator layer may include acids and may be referred to as an acid providing layer or an acid delivery layer in this specification. The nitric oxide source layer/the nitrite delivery layer/the nitrite providing layer and the activator layer/the acid providing layer may be collectively or individually referred to as nitric oxide generating layer(s) in this specification.

### Nitric Oxide Dressing Materials and Construction

As will be understood by one of skill in the art, the materials and dressing constructions described above in relation to the nitric oxide delivery dressings 1200 of Figures 4-5 and elsewhere in the specification may include multiple suitable constructions and different types of materials. For example, the topmost layer furthest away from the wound may be a top or cover film layer, such as a top or cover layer disclosed herein, such as polyurethane materials. Such a top or cover film may be construction from materials used in the cover layer of the RENASYS drape, sold by Smith + Nephew. For example, in some embodiments the cover layer can be an IV3000 top film. Below the top or cover film layer may be a masking or fabric layer, which may be constructed of any suitable material disclosed as a masking or fabric layer herein. The masking layer may be constructed from a stretch and non-stretch polyester, polyethylene, polypropylene, polypropylethylene, and nonwovens and suitable blends constructed thereof. For example, in some embodiments the masking layer can be a 17 gsm polypropylene mask layer. Further suitable nonwovens and blend may also be utilized. In certain embodiments, the masking layer may be foam. Beneath the masking or fabric layer is an activator layer, similar to the activator layers described herein and throughout the specification. Such an activator layer may be constructed from a hydrogel adhesive, optionally containing a central polyester supporting mesh and/or supporting release liners. For example, in some embodiments the activator layer can be a DURAFIBER format loaded with a hydrogel as described herein. In such an example, depending on the dressing size, an equivalent of about 6 grams of hydrogel can be loaded onto a 10.8 cm x 10.8 cm piece of DURAFIBER, which can be cut down to 10 cm x 10 cm resulting in about 5.14 grams of the hydrogel on the 10 cm x 10 cm piece of DURAFIBER. The activator layer may be constructed from any suitable hydrogel material disclosed herein such as an acrylic acid hydrogel and/or a sulfonic acid hydrogel. The activator layer may comprise an absorbent layer 14004 and gel material or gel layer 14006 as described with reference to Figures 12A-I below. Below the activator layer may be an acquisition distribution layer, which may be constructed of any suitable acquisition distribution layer materials disclosed herein, such as in relation to Figure 2. For example, the acquisition distribution layer may be constructed from 3-D knit, gauze and/or stretch polyester fibers woven into a net format, similar to the material used in Acticoat Flex by Smith + Nephew, although silver is optional. In some embodiments the acquisition distribution layer may be constructed from a pre-polymer solution with a mixture of water, surfactant, and polyethylene glycol such as foams used in Allevyn foam by Smith + Nephew. The masking layer and acquisition distribution layer may use the same materials and be interchangeable. In certain embodiments, the acquisition distribution layer may be pressed into the activator layer and/or cured into the activator layer. Curing the acquisition distribution layer into the activator layer may increase the rate of nitric oxide formation due to more rapid transport. Under the acquisition distribution layer, there may be a wound contact layer which may be constructed from any suitable material disclosed herein, such as in relation to Figure 2. For example, the wound contact layer may include a silicone adhesive and perforated polyurethane film. The wound contact layer may include an acrylic adhesive. Alternatively, the wound contact layer may comprise an absorbent layer 14004 and gel material or gel layer 14006 as described with reference to Figures 12A-Ibelow, wherein the wound contact layer operates as another activator layer. A nitric oxide source layer, such as a nitrite layer, constructed from any suitable materials disclosed herein, may be positioned beneath the wound contact layer such that the nitric oxide source layer is directly against a wound or other tissue. In some embodiments, the nitric oxide source layer may be in other positions, such as above the activator layer and/or elsewhere in the dressing. For example, in some embodiments the nitrite providing layer can be a separate 17 gsm polypropylene mesh saturated with a sodium nitrite solution. In certain, embodiments the ALLEVYN or PICO dressings disclosed in Figures 2-3 may be placed directly over an activator layer and underlying nitric oxide source layer. Placing the nitric oxide source layer directly against the wound, periwound area, and/or other tissues may allow for increased release of nitric oxide directly into the tissue.

### Chemiluminescence

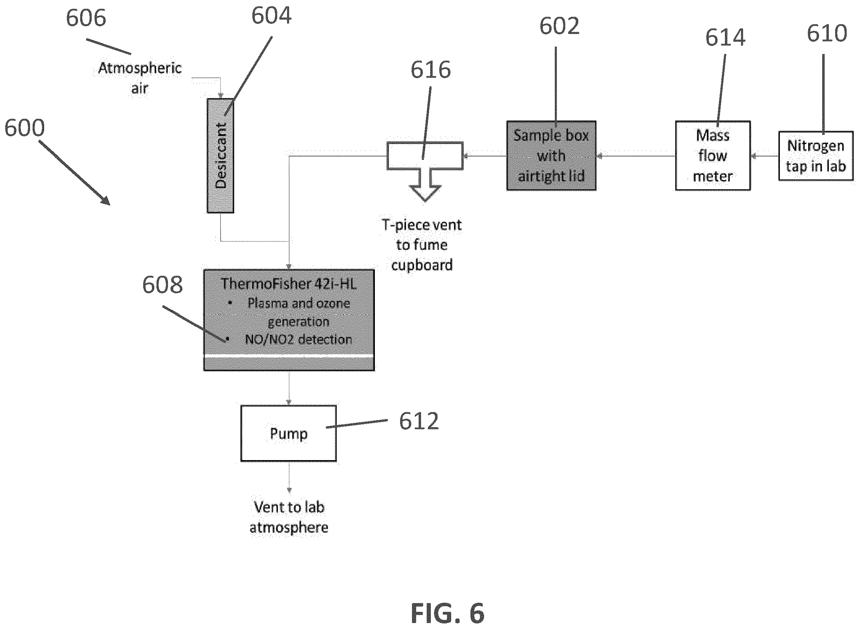

Figure 6 shows an example setup 600 for a chemiluminescence protocol for testing a nitric oxide delivery dressing such as disclosed above in relation to Figures 4 and 5. The protocol may include a sample 602, desiccant 604, an atmospheric air source 606, a chemiluminescence detector 608, a nitrogen supply 610, an air pump 612, a mass flow meter 614, and T-piece connector 616. In certain embodiments, a ThermoFisher 42i-HL detector may be used as a chemiluminescence detector 608. After warming up the equipment with air flow under atmospheric pressure, the sample box 602 and nitrogen supply can be connected to the equipment. The nitrogen flow through the mass flow controller may be set to a suitable value, such as between about: 1 to 100, 10 to 90, 25 to 75, 40 to 60, or about 50 mL/min. After flushing the system (such as for about 1 to 60, 10 to 50, 20 to 40, or about 30 minutes), a nitric oxide source layer (such as a nitrite mesh) and activator layer (such as an acid providing hydrogel) may be placed in the sample chamber 602. In embodiments, the nitrite mesh is smaller in total area than the activator layer. In particular embodiments, the nitric oxide source layer and/or the activator layer may have a length and/or a width of about .5 to 20, 1 to 10, 2 to 8, or about 4 to 6 centimeters. In certain embodiments, the nitric oxide source layer may be 2.5 cm x 2.5 cm while the activator layer is 3 cm x 3 cm.

NO/NO₂ release concentrations may be measured by the chemiluminescence detector at an appropriate rate, checking the concentrations in ppb or ppm and monitoring periodically, such as about every 1, 2, 5, 10, 30, 60 or 90 seconds. In certain embodiments, the NO/NO₂ concentration may be checked in ppm.

As will be understood by one of skill in the art, maximizing NO over NO₂ is desirable for the dressings disclosed herein, such as the dressings described in relation to Figures 4-5. While nitrogen dioxide (NO₂) may exert antimicrobial properties, NO₂ does not have the vasodilating properties nor the capability of activating cell proliferation of NO. It is therefore generally desirable to reduce the generation of NO₂ as far as possible in the acidification of nitrites such as by such means as reducing the oxidation of dissolved nitric oxide (NO) by removing the oxygen from the body of the hydrogel where the acidification of nitrite takes place. The nitric oxide delivery dressings disclosed herein may produce both NO and NO₂. In some embodiments, the nitric oxide dressings disclosed herein may produce NO and NO₂ in a ratio of NO/ NO₂ such as about 0.5:1 to 500:1, 1:1 to 400:1, 10:1 to 300:1, 20:1 to 200:1. 50:1 to 100:1. For example, the ratio may be about or at least about 0.5:1 1.01:1, 1.1:1, 1:1, 2:1. 5:1, 10:1, 20:1, 30:1, 50:1, 100:1, 200:1, or 500:1.

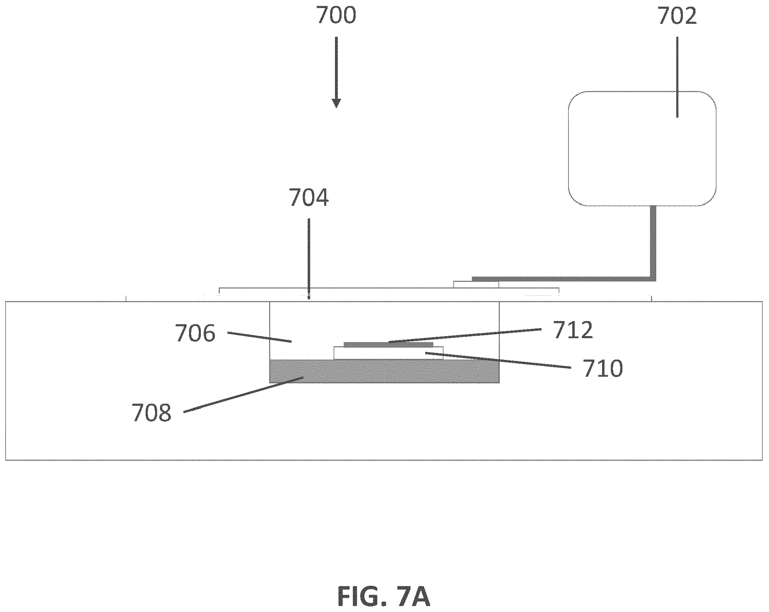

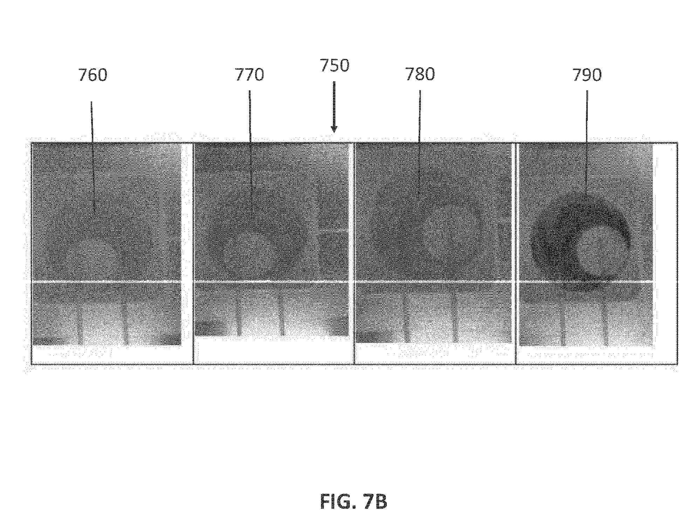

Figures 7A-B show an example of an experimental set-up 700 and the subsequent results 750 demonstrating nitric oxide delivery from a combination of activator layer and nitric oxide source layer, similar to the dressings described in relation to Figures 4 and 5, while under negative pressure. As shown in Figure 7A, a negative pressure wound therapy pump 702 is connected to a negative pressure wound therapy dressing 704 such as described herein in Figures 2A-2D. The dressing is sealed over a chamber 706 containing nitrite test solution 708 which changes color in the presence of NO. Figure 7B shows an example of results of the negative pressure nitric oxide experiment shown in Figure 7A. Prior to applying negative pressure, the test solution did not change color 750. After running negative pressure for a period of time to ensure that no background color change occurred as shown in 760, an activator layer 710 such as described herein (such as an acid-providing hydrogel), was placed in the chamber and negative pressure was applied. Again, no color change occurred 770. Lastly, a nitric oxide source layer 712 such as described herein (such as a sodium nitrite mesh) was placed onto the activator layer 780 without having the nitric oxide source layer touch the nitrite test solution, and negative pressure was applied. After 15 minutes of negative pressure, the indicator solution changed color 790, thereby demonstrating that interaction between the activator layer and the nitric oxide layer can produce nitric oxide, even while under negative pressure.

As will be understood by one of skill in the art, negative pressure may be applied to any of the nitric oxide delivering dressings disclosed herein, such as the dressings described in Figures 4-5 and elsewhere in the specification. A dressing, such as the dressings described in Figures 2A-2D may be placed over an activator layer and nitric oxide source layer which are placed in a wound, thereby delivering nitric oxide to a wound while simultaneously applying negative pressure wound therapy.

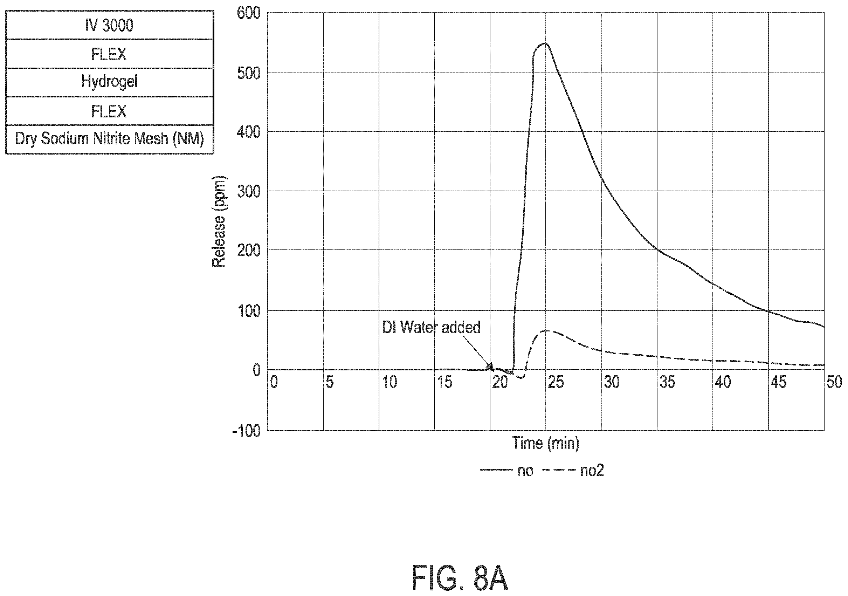

Figures 8A through 8C show examples of chemiluminescence experimental runs using a protocol similar to that described above. As will be understood by one of skill in the art, these measurements taken in these experimental runs are merely exemplary and the disclosures herein are not limited to such values. Figure 8A shows the experimental results when testing a dry sodium nitrate mesh embodiment with the arrangement shown in Figure 8A, including a polyurethane cover layer overlying a stretch polyester ADL layer, positioned over a hydrogel activator layer sandwiched between another stretch polyester ADL layer over a dry sodium nitrite mesh as shown in the figure. In this experimental run, after the DI water was added, the dry sodium nitrate mesh released approximately 550 ppm NO and 75 ppm NO₂ at its peak at the 25 minute mark, slowly reducing in concentration to approximately 80 ppm NO and 10 ppm NO₂ at the 50 minute mark.

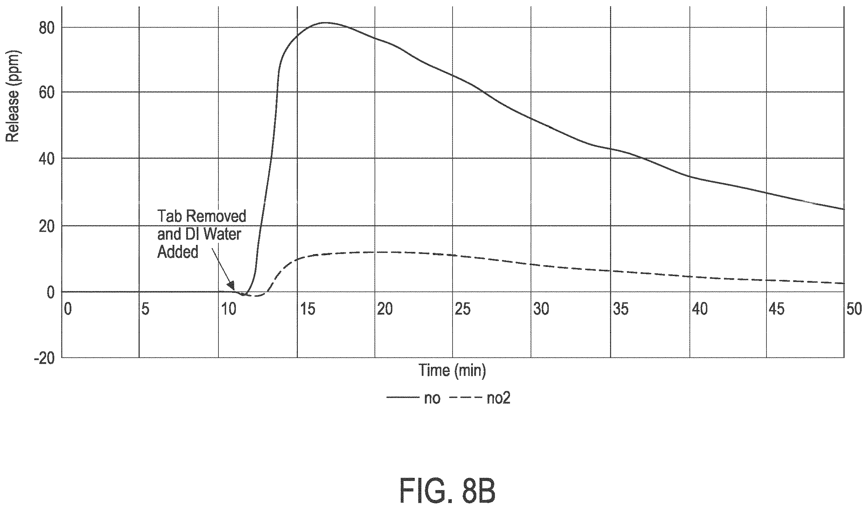

Figure 8B shows the experimental results when testing a full dressing design with a pull-out tab and self-sealing borders. The pull out tab is used to initially separate the nitric oxide source layer from the activator layer, therefore when the tab is removed and the dressing becomes wet, the interaction between the nitric oxide source layer and the activator layer produces nitric oxide. In this experimental run, after the DI water was added, the full dressing design with the pull-out tab and self-sealing borders released approximately 84 ppm NO and 15 ppm NO₂ at its peak at the 17 minute mark, slowly reducing in concentration to approximately 25 ppm NO and 5 ppm NO₂ at the 50 minute mark.

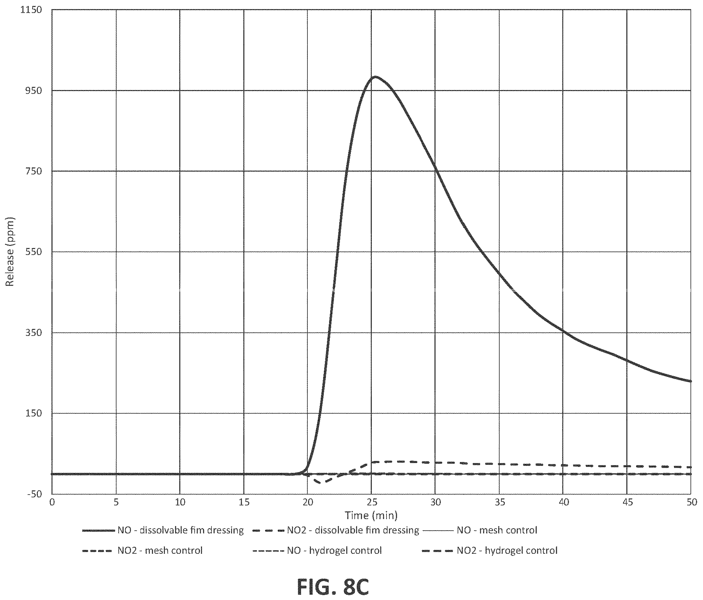

Figure 8C shows an example of the experimental results for a dressing containing a degradable film. Here, a degradable film was placed between the activator layer and the nitric oxide source layer, thereby generating nitric oxide once the degradable layer breaks down. In this experimental run, after the DI water was added, the dressing containing a degradable film released approximately 1000 ppm NO and 45 ppm NO₂ at its peak at the 25 minute mark, slowly reducing in concentration to approximately 225 ppm NO and 20 ppm NO₂ at the 50 minute mark. The experimental protocol was also utilized to test an activator layer containing sodium isoascorbate. In this experimental run, after the DI water was added, the activator layer containing sodium isoascorbate released approximately 52 ppm NO and 4 ppm NO₂ at its first peak at the 80 minute mark, 66 ppm NO and 5 ppm NO₂ at its second and maximum peak at the 110 minute mark slowly reducing in concentration to approximately 45 ppm NO and 2 ppm NO₂ at the 160 minute mark.

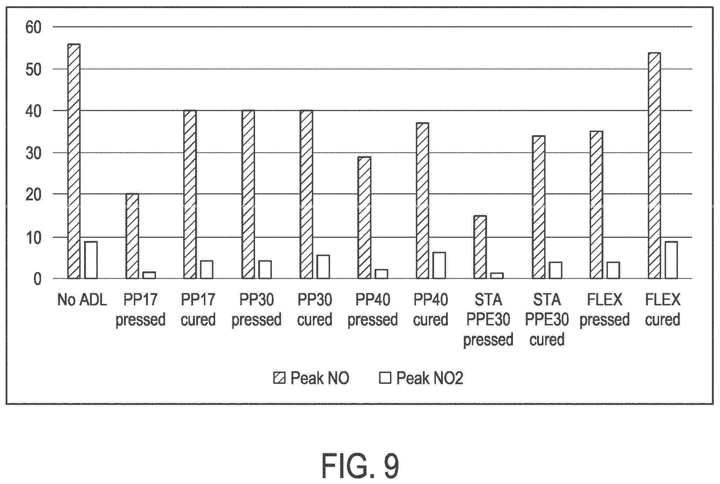

Figure 9 shows an example of the relative peak output in ppm for activator hydrogels (acid providing) either with an acquisition distribution layer or without, including polypropylene, polypropylethylene, or stretch polyester acquisition distribution layers with various gsm (g/m²). With no acquisition distribution layer, the peak NO and NO₂ concentrations were approximately 55 ppm and 10 ppm respectively; however, one of skill in the art will understand that an acquisition distribution layer may allow for improved fluid distribution and handling throughout a larger area such as a dressing. With a 17 gsm polypropylene pressed acquisition distribution layer, the peak NO and NO₂ concentrations were approximately 20 ppm and 2 ppm respectively. With a 17 gsm polypropylene cured acquisition distribution layer, the peak NO and NO₂ concentrations were approximately 40 ppm and 5 ppm respectively. As explained above, curing the acquisition distribution layer may allow for increased fluid transport and an increased rate of nitric oxide formation. With a polypropylene 30 g/m² pressed acquisition distribution layer, the peak NO and NO₂ concentrations were approximately 40 ppm and 5 ppm respectively. With a polypropylene 30 g/m² acquisition distribution layer, the peak NO and NO₂ concentrations were approximately 40 ppm and 5 ppm respectively. With a polypropylene 40 g/m² pressed acquisition distribution layer, the peak NO and NO₂ concentrations were approximately 30 ppm and 2 ppm respectively. With a polypropylene 40 g/m² cured acquisition distribution layer, the peak NO and NO₂ concentrations were approximately 38 ppm and 5 ppm respectively. With a polypropylethylene 30 g/m² pressed acquisition distribution layer, the peak NO and NO₂ concentrations were approximately 35 ppm and 3 ppm respectively. With a polypropylethylene 30 g/m² cured acquisition distribution layer, the peak NO and NO₂ concentrations were approximately 35 ppm and 3 ppm respectively. With a stretch polyester pressed acquisition distribution layer, the peak NO and NO₂ concentrations were approximately 35 ppm and 3 ppm respectively. With a FLEX pressed acquisition distribution layer, the peak NO and NO₂ concentrations were approximately 55 ppm and 8 ppm respectively.

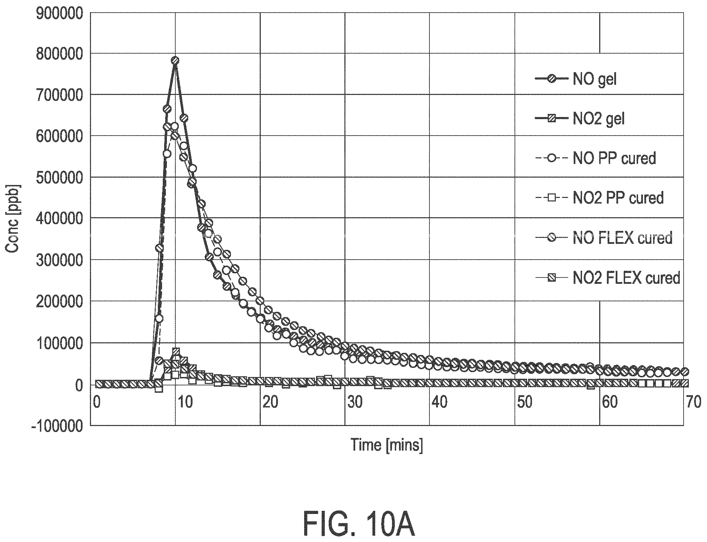

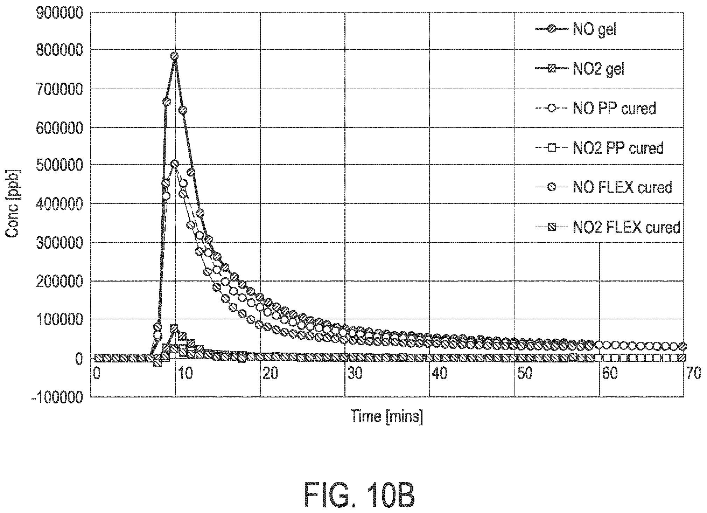

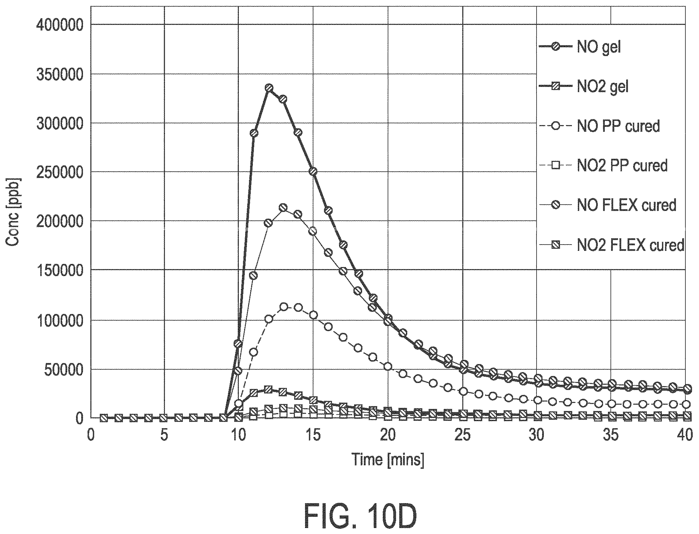

Figures 10A through 10D show examples of the concentration of NO and NO₂ over time for several embodiments incorporating an activator layer and nitric oxide providing layer. As shown in Figures 10A-B, an activator layer containing approximately 2-3% sodium isoascorbate was tested with or without different acquisition distribution layers that were pressed or cured. The gel with no acquisition distribution layer produced (p indicating peak) pNO = 785ppm and pNO2 = 78ppm. The activator layer with a stretch polyester pressed into the gel produced pNO = 506ppm and pNO2 = 24ppm. For stretch polyester cured on the activator layer, pNO = 625ppm; pNO2 = 50ppm. For polypropylene pressed into the gel, the pNO = 508ppm and pNO2 = 26ppm. For polypropylene cured into the gel, the pNO = 624ppm and pNO2 = 26ppm.

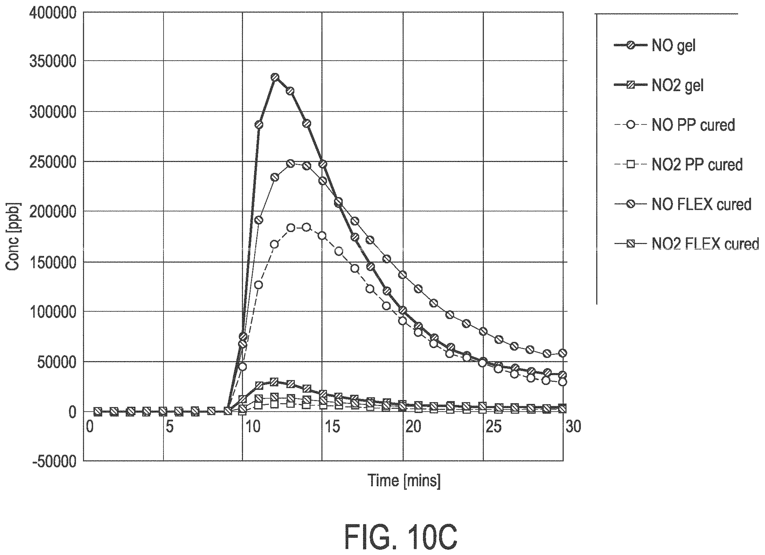

Figures 10C-10D show examples of the concentration of NO and NO₂ over time for an activator layer containing approximately 1-2% sodium isoascorbate with or without different acquisition distribution layers that were pressed or cured. The activator layer with no ADL produced pNO = 334ppm; pNO2 = 40ppm. For the stretch polyester acquisition distribution layer pressed into the activator layer, pNO = 211ppm and pNO2 = 10ppm. For the stretch polyester acquisition distribution layer cured into the activator layer, pNO = 247ppm and pNO2 = 14ppm. For the polypropylene acquisition distribution layer pressed into the activator layer, pNO = 112ppm and pNO2 = 5ppm. For the polypropylene acquisition distribution layer cured into the activator layer, pNO = 184ppm and pNO2 = 8ppm. As explained elsewhere in the specification, curing an acquisition distribution layer into an activator layer may improve fluid handling and nitric oxide production relative to nitrogen dioxide production.

### Xerogels and Hydrogel Construction

Reference may be made throughout the specification to xerogels. A xerogel may be formed from a gel by drying with unhindered shrinkage. As will be understood by one of skill in the art, a xerogel is a gel that has very low free water content, so low that minimal reaction to form nitric oxide will occur without the addition of further water and/or liquid. For example, a xerogel may be substantially free of water in the dry state. Drying may be completed by any suitable means known in the art (e.g., freeze drying).

In certain examples, hydrogels (which may subsequently become xerogels after drying) may be generated with or without glycerol, and may contain a standard amount or double, triple, or quaruple the required amount of crosslinker PEG diacrylate as needed. A 2-Acrylamido-2-methyl-1-propanesulfonic acid sodium salt solution may be present in the xerogel. Hydrogels and xerogels may be created by converting 2-acrylamido-2-methyl-1-propanesulphonic acid (SA), stabilised with MEHQ as supplied, to a sodium salt by dissolving into water, then neutralising with 50% NaOH to pH 7.0 with cooling from a 10C water bath to form a solution of the neutralised acid (NaAMPS). Hydrogels can contain about 5.393 wt% 2-acrylamido-2-methyl-1-propanesulphonic acid (equating to 1.0 SA), about 4.654 wt% 2-acrylamido-2-methyl-1-propanesulphonic acid (equating to 0.85 SA), about 2.839 wt% 2-acrylamido-2-methyl-1-propanesulphonic acid (equating to 0.5 SA), and/or a range of between about 1.457 wt% 2-acrylamido-2-methyl-1-propanesulphonic acid (equating to 0.25 SA) to about 7.704 wt% 2-acrylamido-2-methyl-1-propanesulphonic acid (equating to 1.5 SA). Hydrogel prepolymers may be prepared by predispersing 2-hydroxy-2-methylpropiophenone photoinitiator into PEG diacrylate under minimal light, then mixing for 10-20 mins with a mixture of 58% aqueous sodium 2-acrylamido-2-methyl-1-propanesulfonate solution (Na AMPS), (Sodium iso-ascorbate, preground 2-Acrylamido-2-methyl-1-propanesulfonic acid (AMPS acid) and glycerol. The AMPS acid may be fully dissolved in the stirred Na AMPS solution prior to slowly adding the glycerol, and then the photoinitiator/diacrylate mixture in a water bath. In certain embodiments, hydrogels may also prepared with twice the normal amount of photoinitiator/crosslinker and/or the omission of glycerol and/or using triple the amounts of prepolymer mix in the molds to form gels with three times the thickness.

### Nitric Oxide Generating Dressings Utilizing Dry Sodium Nitrite

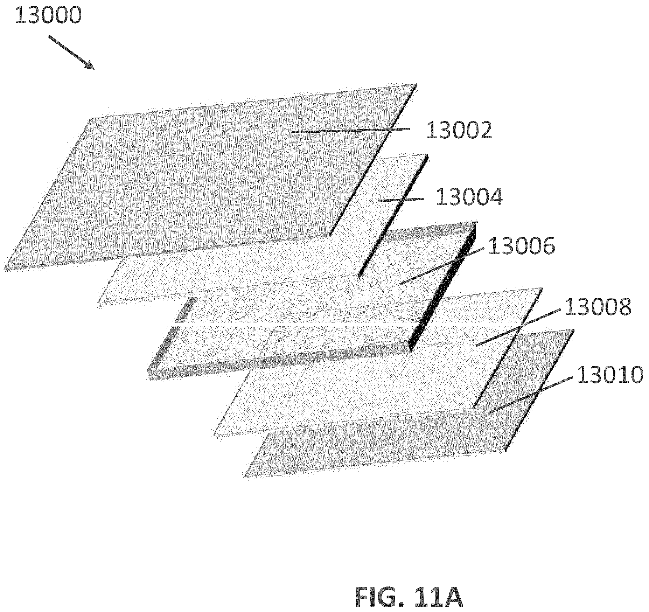

Figures 11A-11D depict embodiments of a nitric oxide generating wound dressing with various arrangements of layers. One of skill in the art will understand that the various layers depicted in Figures 11A-11D may be ordered in any suitable order and the orders depicted in the figures are merely examples. In some embodiments, the uppermost layer may be a cover layer 13002, which may have any of the same features, materials, or other details of cover layers disclosed herein, such as being constructed from a film. Said cover layer 13002 may be suitable for sealing a dressing over a wound and for connecting to a source of negative pressure and/or for maintaining negative pressure at a wound site. In certain embodiments, the border region of the cover layer 13002 may be attached to the skin around the wound, forming a seal, such that the wound exudate can be contained within the wound dressing 13000. Below the cover layer, there may be a masking or obscuring layer 13004 (heretofore referred to as a "masking layer") to prevent or limit visualization of the wound or the wound exudate through the cover layer 13002. The masking layer 13004 may be positioned beneath at least a portion of the cover layer 13002. In some embodiments, the masking layer 13004 can have any of the same features, materials, or other details of any of the other embodiments of the masking layers disclosed herein, including but not limited to having any viewing windows or holes. Examples of wound dressings with obscuring layers and viewing windows are described in International Patent Publications WO2013/007973 and WO2014/020440. Additionally, the masking layer 13004 may be positioned adjacent to the cover layer, or can be positioned adjacent to any other dressing layer as desired. In the illustrated embodiment, the masking layer 13004 is positioned between the cover layer 13002 and the activator layer 13006. The activator layer 13006 may comprise an absorbent layer 14004 and gel material or gel layer 14006 as described with reference to Figures 12A-I below. As explained elsewhere herein and as will be understood by one of skill in the art, the activator layer may be an acid providing layer or other suitable layer. In certain embodiments, the masking layer 13004 can be adhered to or integrally formed with the cover layer 13002. The masking layer 13004 may be configured to have approximately the same size and shape as the activator layer 13006 so as to overlay it. The masking layer 13004 may be of a smaller area than the cover layer 13002. In certain embodiments, the masking layer 13004 can horizontally wick fluid and may function as an acquisition distribution layer as well.

In particular embodiments, the activator layer 13006 may have any of the same features, materials, or other details of any of the other embodiments of activator layers disclosed herein. For example, the activator layer 13006 may be adhesive and may be constructed of a hydrogel or xerogel configured to have a plurality of acidic groups or moieties which may provide protons in an aqueous environment. As explained elsewhere in the specification, under such acidic conditions nitrite ions from a nitric oxide source layer 13010 may be reduced to nitric oxide for delivery to a wound or intact skin. As explained elsewhere herein and as will be understood by one of skill in the art, the activator layer may be a nitrite providing layer or other suitable layer. The activator layer 13006 (e.g. hydrogel layer) may include a plurality of perforations that extend through the thickness of the activator layer, as described elsewhere herein. The plurality of perforations may allow or facilitate passage of wound exudate through the activator layer, such that wound exudate below or around the activator layer can be transported to one or more additional absorbing layers and/or an evaporative layer or layers (e.g. cover layer) above the activator layer, thus preventing excessive buildup of wound exudate below the activator layer 13006 Additionally, the plurality of perforations may provide increased surface area of the activator layer, thereby increasing the absorption rate of the activator layer.

As shown in Figure 11A, in embodiments, an acquisition distribution layer 13008 may be placed between the activator layer 13006 and the nitrite providing layer 13010. In certain embodiments, the acquisition distribution layer 13008 may be constructed so as to advantageously horizontally wick fluid, such as wound exudate, as it is absorbed through the layers of the dressing 13000. Such lateral wicking of fluid may allow maximum distribution of the fluid through the activator layer 13006, enabling the activator layer 13006 to reach its full holding capacity. Further, acquisition distribution layer 13008 may facilitate nitric oxide production, as nitrite ion dissolved in fluid may spread across the surface of the activator layer 13006 more quickly. Some embodiments of the acquisition distribution layer 13008 may comprise viscose, polyester, polypropylene, cellulose, or a combination of some or all of these, and the material may be needle-punched. Some embodiments of the acquisition distribution layer 13008 may comprise cellulose in the range of 40-160 gsm (or about 40 to about 160 gsm), for example 80 (or about 80) gsm. Some embodiments of the acquisition distribution layer 13008 may comprise polyethylene in the range of 40-150 grams per square meter (gsm). In some embodiments, the acquisition distribution layer 13008 may have a thickness of 1.2 mm or about 1.2 mm, or may have a thickness in the range of about 0.5 mm to 3.0 mm, about 0.5 mm to about 3.0 mm., 0.7 mm to 2.5 mm, 0.9 mm to 2.1 mm, or 1.1 mm to 1.5 mm. In certain embodiments, the acquisition distribution layer 13008 may be constructed from a material which resists compression under the levels of negative pressure commonly applied during negative pressure therapy.

The acquisition distribution layer 13008 may include a plurality of loosely packed fibers, which may be arranged in a substantially horizontal fibrous network. In some embodiments, the acquisition distribution layer 13008 may consist of a mix of two fiber types. One may be a flat fiber which may be 20 µm to 50 µm in width, or approximately 20 µm to approximately 50 µm in width, and may comprise a cellulosic based material. The other fiber may be a two component fiber that has an inner core that is 8 µm to 10 µm in diameter, approximately is 8 µm to approximately 10 µm in diameter, 7 µm to 11 µm in diameter, 6 µm to 12 µm in diameter, or 5 µm to 13 µm in diameter and an outer layer with a thickness of 1 µm to 2 µm, approximately 1 µm to approximately 2 µm, 1 µm to 2.3 µm, 0.8 µm to 2.5 µm, or 0.5 µm to 3 µm. The two component fiber may be a mix of a polyethylene (PE) type material, and polyethylene terephthalate (PET). In some embodiments the inner core of the two component fiber may be PET and the outer layer may be PE. The PE/PET fibers may have a smooth surface morphology, while the cellulosic fibers may have a relatively rougher surface morphology. In some embodiments the ADL material may comprise about 60% to about 90% cellulosic fibers, for example approximately 75% cellulosic fibers, and may comprise about 10% to about 40% PE/PET fibers, for example approximately 25% PE/PET fibers. In some embodiments, the acquisition distribution layer 13004 may include split microfibers.

A majority of the fiber volume may extend horizontally (that is, parallel to the plane of the top and bottom surfaces of the material), or substantially or generally horizontally. In another embodiment, 80%-90% (or approximately 80% to approximately 90%) or more of the fiber volume may extend horizontally, or substantially or generally horizontally. In another embodiment, all or substantially all of the fiber volume may extend horizontally, or substantially or generally horizontally. In some embodiments, a majority, 80%-90% (or approximately 80% to approximately 90%) of the fibers or more, or even all or substantially all of the fibers, span a distance perpendicular to the thickness of the acquisition distribution layer 13004 (a horizontal or lateral distance) that is greater than the thickness of the acquisition distribution layer 13004. In some embodiments, the horizontal or lateral distance spanned by such fibers is 2 times (or about 2 times) or more, 3 times (or about 3 times) or more, 4 times (or about 4 times) or more, 5 times (or about 5 times) or more, or 10 times (or about 10 times) or more the thickness of the acquisition distribution layer 13008. The orientation of such fibers may promote lateral wicking of fluid through the acquisition distribution layer 13008. This may more evenly distribute fluid such as wound exudate throughout the acquisition distribution layer 13008. In some embodiments, the ratio of the amount of fluid wicked laterally across the acquisition distribution layer 13008 to the amount of fluid wicked vertically through the acquisition distribution layer 13004 under negative pressure may be 2:1 or more, or approximately 2:1 or more, or may be up to 10:1 or more, or approximately 10:1 or more, in some embodiments.

Continuing with Figure 11A, in embodiments a nitric oxide source layer 13010 may be provided beneath the acquisition distribution layer 13004. Such a nitric oxide source layer 13010 may have any of the same features, materials, or other details of any of the other embodiments of nitric oxide source layers disclosed herein, for instance, the nitric oxide source layer 13010 may be a nitrite providing layer. For example, the nitric oxide source layer may be a wet mesh imbued with sodium nitrite solution. In some embodiments, the nitric oxide source layer 13010 may be dry and include a dry nitrite source, such as dry sodium nitrite. Such dry sodium nitrite may be loaded into a material layer, said material layer constructed from a suitable material such as any material disclosed herein. As will be understood by one of skill in the art, a dry material and/or substance is one that is free or relatively free of liquid. For example, polypropylene, polyethylene, or melt extrudable fibers may be suitable materials for such a layer. In embodiments, such a nitric oxide source layer 13010 layer may need to be separated initially from the activator layer 13006 when the activator layer is a hydrogel so as to avoid reaction and production of nitric oxide prior to application to a wound and/or skin. As depicted in Figure 11A a dry fluid acquisition layer 13008 may serve to separate the nitric oxide source layer 13010 and a hydrogel activator layer 13006 prior to application. However, such a dry sodium nitrite providing layer may be adjacent to a xerogel activator layer 13006, since a xerogel will not be wet. In the instance of a xerogel, activation may occur upon contact with fluid such as wound exudate as the wound exudate wicks through the dressing. In the instance of a hydrogel, once fluid such as wound exudate comes into contact with the acquisition distribution layer 13008, then nitrite ions may come into contact with the acidic environment created by the activator layer, thereby generating nitric oxide which may then migrate into the wound and/or skin. In some embodiments, each of the layers such as the nitric oxide source layer, the activator layer, and any other suitable layer may be stored dry prior to use. Prior to application to the skin or wound, the layers may be wet by a suitable liquid such as saline.

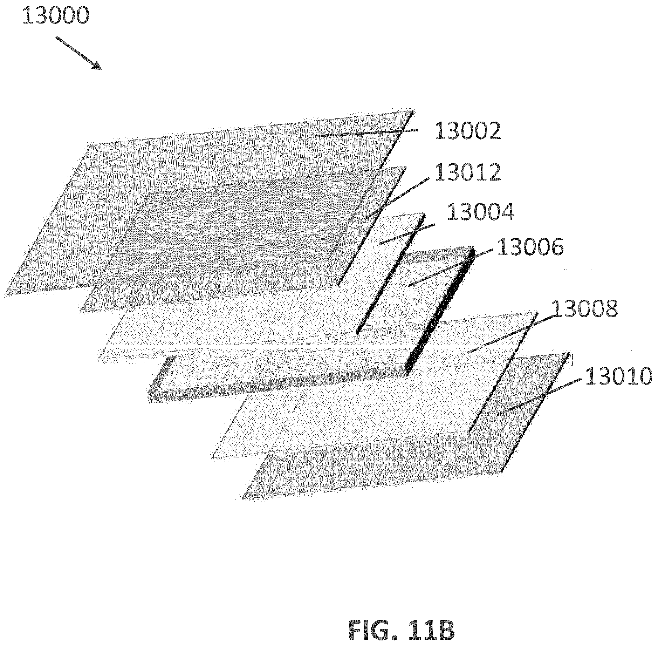

As depicted in Figure 11B, to maintain nitric oxide release there could be a number of layers containing dry sodium nitrite, for example a first nitric oxide source layer 13010 and a second nitric oxide source layer 13012 that would be 'activated' as wound fluid reaches and wets out the layer(s), enabling the sodium nitrite to come into contact with the acidic groups of the hydrogel or xerogel of activator layer 13006, thereby producing nitric oxide. In certain embodiments, there may be 2, 3, 4, 5, 6 or more layers containing dry sodium nitrite. As shown in Figure 11B, the masking layer 13004 may serve to prevent contact between the second nitric oxide source layer 13012 and the activator layer 13006. In certain embodiments, additional acquisition distribution layers and/or masking layers may be sandwiched with activator layers to provide additional sources of nitric oxide.

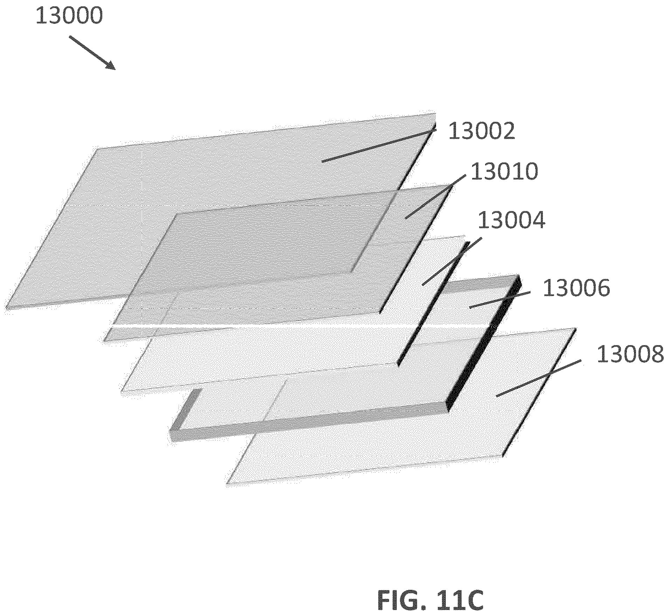

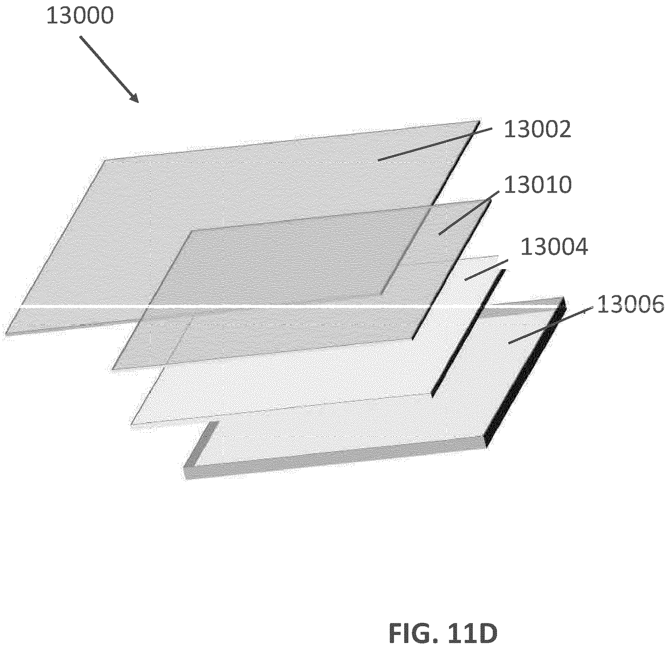

As depicted in Figures 11C-11D, in embodiments the activator layer 13006 may be positioned beneath the nitric oxide source layer, thereby relying on the dressing wetting out (such as from wound exudate) and activating the nitrite providing layer 13010. A masking layer or acquisition distribution layer 13004 may be provided between the activator layer 13006 and the nitrite providing layer 13010. Figure 11C illustrates the activator layer 13006 being positioned above an acquisition distribution layer 13008, and Figure 11D illustrates the activator layer being the lowermost layer of the wound dressing. The activator layer 13006 of these embodiments may comprise an absorbent layer 14004 and gel material or gel layer 14006 as described with reference to Figures 12A-I below

### Wound Dressings with Active Wound Contact Layers

Figures 12A-12I depict embodiments of wound dressings with a gel material used as a wound contact layer, thereby forming an active wound contact layer that may be utilized in embodiments of wound dressings for generating nitric oxide as described above. The gel material may comprise any of the hydrogel or xerogel materials described herein. For example, the gel material may be a hydrogel monomer solution (i.e., 0.7 SA with 1.4% sodium isoascorbate). SA indicates a 2-Acrylamido-2-methyl-1-propanesulfonic acid. The gel material may comprise acidic groups or moeites. The gel material may comprise acidic groups that are attached to the polymer structure of the gel material. The gel structure may comprise cross linked 2-Acrylamido-2-methyl-1-propanesulfonic acid (with pendant sulphonic acid groups) and a neutralized version, e.g. Na salt (Sodium 2-Acrylamido-2-methyl-1-propanesulfonate) and a cross linker to form a cross linked polymer after polymerization

The wound dressings described herein are used with a nitric oxide generating layer or nitrite providing layer (e.g., 12600 or 13010) as described above. The materials and the order of the layers described with respect to a specific figure or embodiment may apply to any of the embodiments described herein. As depicted in Figure 12A, a wound dressing 14000 may include a cover layer or top film 14002, an absorbent layer 14004 and a gel material or gel layer 14006. The wound dressing 14000 further includes one or more nitrite providing layers (e.g., 12600 or 13010) and may further include other layers, not shown, in any of the arrangements described above. In some embodiments, the wound dressing 14000 may include an acquisition distribution layer (e.g., 13008). The cover layer or top film 14002 may be positioned above the absorbent layer 14004. The gel material or gel layer 14006 may be positioned below the absorbent layer 14004. The nitrite providing layer(s) (e.g., 12600 or 13010) may be positioned above the absorbent layer 14004, between the gel material or gel layer 14006 and the absorbent layer 14004, beneath the gel material or gel layer 14006, beneath the cover layer 14002, or between the cover layer 14002 and the absorbent layer 14004. In embodiments having an acquisition distribution layer (e.g., 13008) the acquisition distribution layer may be positioned between the one or more nitrite providing layers and the gel material or gel layer 14006, the cover layer 14002 and the absorbent layer 14004 or between the absorbent layer 14004 and the gel material or gel layer 14006. In some embodiments, the acquisition distribution layer may act as the absorbent layer 14004. The absorbent layer 14004 may be a foam, for example ALLEVYN^{™} foam, foam, or a nonwoven material. The gel material or gel layer 14006 may be a hydrogel or a xerogel as described elsewhere in this specification.

In some embodiments the nitrite providing layer (e.g., 12600 or 13010) may be prepared and provided separate from the wound dressing 14000. For example, the nitrite providing layer (e.g., 12600 or 13010) may be applied to the wound and the wound dressing 14000 may be applied above the nitrite providing layer (e.g., 12600 or 13010).

As depicted in Figure 12A, the gel material or gel layer 14006 may be perforated in a pattern to define a plurality of fluid channels. For the purposes of this disclosure, perforated or perforation(s) or any variation herein, is used to describe any kind of opening, hole, or aperture, regardless of how it is formed in any of the materials or layers described herein. The plurality of fluid channels will allow a liquid removed from a wound to pass through the gel material or gel layer 14006 and to the absorbent layer 14004. The perforations may be about 1 mm to about 8 mm in diameter, for example, 1 mm, 2 mm, 3 mm, 4 mm, 5 mm, 6 mm, 7 mm, 8 mm, or any value in between. The percentage of open area (i.e., the area of the perforations) of the gel layer 14006 as compared to non-open area (i.e., the area of the gel material or gel layer) may be about 3% open area to about 60% open area, for example, 5% open area, 10% open area, 20% open area, 30% open area, 40% open area, 50% open area, 60% open area, and any value in between.

The gel material or gel layer 14006 may be adhered to a wound-facing surface of the absorbent layer 14004 as described in more detail below. The gel material or gel layer 14006 may have a thickness of about .1 mm or greater. The absorbent layer 14004 may have a thickness of about 3 mm to about 6 mm, for example 3 mm, 4 mm, 5 mm, 6 mm or any value in between. The gel material or gel layer 14006 may be configured to provide acid to one or more nitrite providing layers (e.g., 12600 or 13010) to release nitric oxide.

In some embodiments, a perforated mesh material 14008 may be wetted with the gel material or gel layer 14006 before adhering the gel material or gel layer 14006 to the absorbent layer 14004. The gel material or gel layer 14006 may be applied to the perforated mesh material 14008 to saturate or wet the mesh material 14008. Depending on the amount of gel material 14006 applied to the perforated mesh material 14008, the gel material 14006 may need to be manipulated to evenly spread the gel material 14006 over the perforated mesh material 14008. The gel material or gel layer 14006 will not fill the perforations of the mesh 14008. The wetted or saturated perforated mesh material 14008 may be transferred or applied to the absorbent layer 14004. The act of transferring or applying the wetted perforated mesh material 14008 may allow any gel material 14006 in the perforations to be separated from the perforations, meaning the perforations will not be blocked by the gel material 14006. The perforations of the perforated mesh material 14008 and the perforations of the gel material or gel layer 14006 may be aligned. The perforated mesh material 14008 may be cured to the absorbent layer 14004, for example, by UV light or heat. The perforated mesh material 14008 may be a 17 gsm pre-perforated polypropylene mesh.

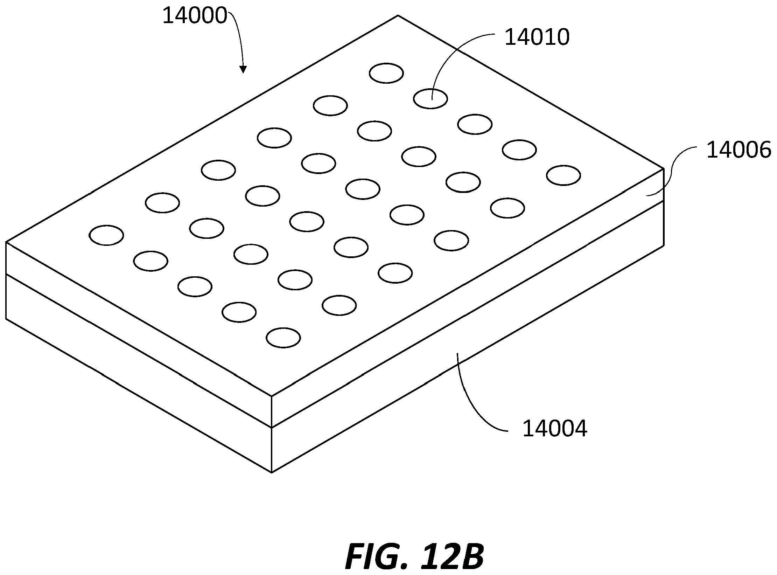

Figure 12B shows a bottom perspective view of the wound dressing 14000 of Figure 12A with the cover layer 14002 not shown. The gel material or gel layer 14006 may include perforations 14010 creating fluid channels to the absorbent layer 14004. The perforations 14010 may be positioned in a pattern. For example, the perforations 14010 may be arranged in lines with the perforations 14010 equally spaced and positioned next to each other (e.g., as shown). In some embodiments, the perforations 14010 may be positioned in a checkerboard type pattern with parallel lines and rows of perforations, or perforations maybe offset or staggered relative to each neighboring perforation 14010. The perforations 14010 may have any shape outer perimeter, for example, circular, square, rectangular, triangular, diamond, etc. The perforations 14010 may have different dimensions as they extend through the material or layer. For example, the perforations 14010 may have an overall pyramid shape. Described another way the perforations 14010 may increase or decrease in cross-sectional area as they extend through the layer or material.

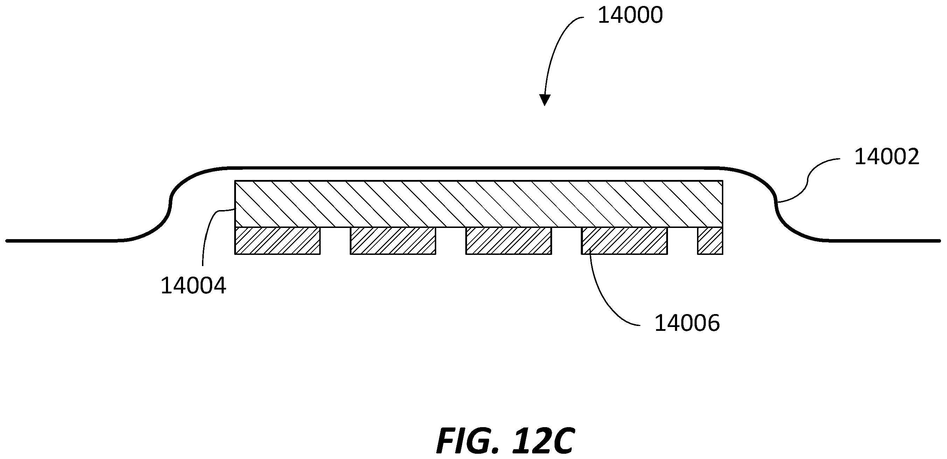

Figure 12C shows another embodiment of the wound dressing 14000. The wound dressing 14000 may include the cover layer 14002, the absorbent layer 14004, and the gel material or gel layer 14006. The cover layer 14002 may be placed above the absorbent layer 14004 and the gel material or gel layer 14006 may be adhered to the wound-facing surface of the absorbent layer 14004. The wound dressing 14000 of Figure 12C may include one or more nitrite providing layers (e.g., 12600 or 13010). In some embodiments, the wound dressing 14000 may include an acquisition distribution layer (e.g., 13008). The nitrite providing layer(s) (e.g., 12600 or 13010) may be positioned above the absorbent layer 14004, between the gel material or gel layer 14006 and the absorbent layer 14004, beneath the gel material or gel layer 14006, beneath the cover layer 14002, or between the cover layer 14002 and the absorbent layer 14004. In embodiments having an acquisition distribution layer (e.g., 13008), the acquisition distribution layer may be placed between the one or more nitrite providing layers and the gel material or gel layer 14006, the cover layer 14002 and the absorbent layer 14004 or between the absorbent layer 14004 and the gel material or gel layer 14006.

In some embodiments the nitrite providing layer (e.g., 12600 or 13010) may be prepared and provided separate from the wound dressing 14000. For example, the nitrite providing layer (e.g., 12600 or 13010) may be applied to the wound and the wound dressing 14000 may be applied above the nitrite providing layer (e.g., 12600 or 13010).

The gel material or gel layer 14006 may be cured in a patterned mold to create a pattern of perforations in the molded gel layer. Any mold that may create the desired perforations in the gel material or gel layer 14006 is suitable for use. The gel material or gel layer 14006 may be applied to the mold and then at least partially cured, for example, via the use of UV light or heat. After the gel material or gel layer 14006 is at least partially cured, the absorbent layer 14004 may be applied to the mold and pushed against the at least partially cured gel material or gel layer 14006 to adhere the gel material or gel layer 14006 to the absorbent layer 14004. It is beneficial to at least partially cure the gel material 14004 prior to applying the absorbent layer 14004 because, UV light for example, cannot pass through the mold or the absorbent layer 14004 to cure the gel material or gel layer 14006. In some manufacturing methods, if heat is used to cure the gel material or gel layer 14006, it may be possible to cure the gel material or gel layer 14006 while the absorbent layer 14004 is applied to the mold.

In some embodiments, the gel material or gel layer 14006 may be spread across the wound-facing surface of the absorbent layer 14004 and partially cured. A patterned mold may then be applied to the gel material or gel layer 14006 to create the patterned perforations. In some methods, a force may be applied to the patterned mold or foam to help create the overall patterned perforations in the gel material or gel layer 14006.

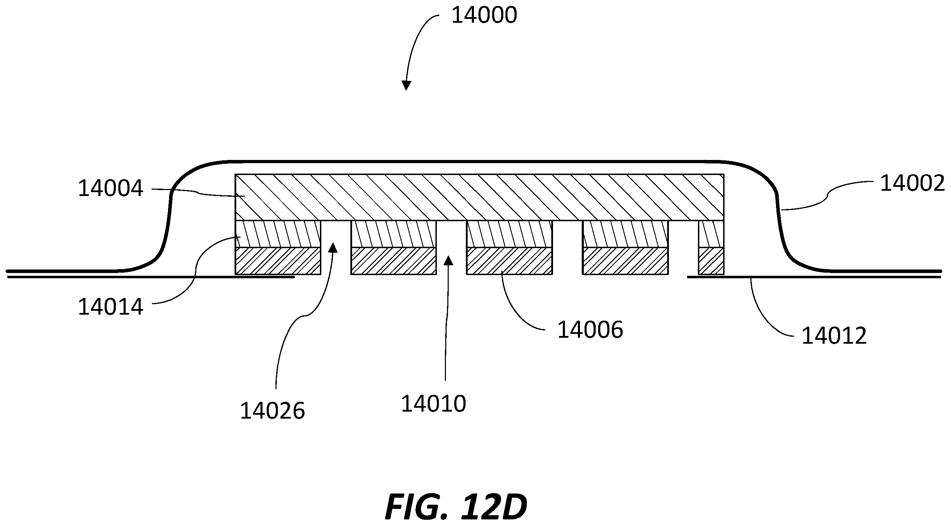

Figure 12D shows another embodiment of a wound dressing 14000. The wound dressing 14000 may include the cover layer 14002, the absorbent layer 14004, a perforated absorbent layer 14014, the gel material or gel layer 14006, and a tissue interface layer 14012. The perforated absorbent layer 14014 may correspond to the perforated gel material or gel layer 14006. For example, the perforations 14026 may align with the perforations 14010. The perforations 14026 may have the same shape and dimensions as the perforations 14010. The perforations 14026 may have different a different shape or dimensions than the perforations 14010. The perforations may be about 1 mm to about 8 mm in diameter, for example, 1 mm, 2 mm, 3 mm, 4 mm, 5 mm, 6 mm, 7 mm, 8 mm, or any value in between. The percentage of open area (i.e., the area of the perforations) of the perforated absorbent layer 14014 as compared to non-open area (i.e., the absorbent portion of the perforated absorbent layer) may be about 3% open area to about 60% open area, for example, 5% open area, 10% open area, 20% open area, 30% open area, 40% open area, 50% open area, 60% open area and any value in between. The perforated absorbent layer 14014 may have a thickness of about 3 mm to about 6 mm, for example 3 mm, 4 mm, 5 mm, 6 mm or any value in between

The perforated absorbent layer 14014 may be the same material as the absorbent layer 14004. The perforated absorbent layer 14014 may be a different material than the absorbent layer 14004. The perforated absorbent layer 14014 may be placed between the cover layer 14002 and the gel material or gel layer 14006. The gel material or gel layer 14006 applied to a lower surface of the perforated absorbent layer, and may be cured or adhered to the perforated absorbent layer 14014. The tissue interface layer 14012 may be placed under the gel material or gel layer 14006.

The wound dressing 14000 of Figure 12D may include one or more nitrite providing layers (e.g., 12600 or 13010). In some embodiments, the wound dressing 14000 may include an acquisition distribution layer (e.g., 13008). The nitrite providing layer(s) (e.g., 12600 or 13010) may be positioned above the absorbent layer 14004, between the absorbent layer 14004 and the perforated absorbent layer 14014, between the gel material or gel layer 14006 and the perforated absorbent layer 14014, beneath the gel material or gel layer 14006, beneath the cover layer 14002, or between the cover layer 14002 and the absorbent layer 14004. In embodiments having an acquisition distribution layer (e.g., 13008), the acquisition distribution layer may be placed between the one or more nitrite providing layers and the gel material or gel layer 14006, between the cover layer 14002 and the absorbent layer 14004 or between the absorbent layer 14004 and the gel material or gel layer 14006.

In some embodiments the nitrite providing layer (e.g., 12600 or 13010) may be prepared and provided separate from the wound dressing 14000. For example, the nitrite providing layer (e.g., 12600 or 13010) may be applied to the wound and the wound dressing 14000 may be applied above the nitrite providing layer (e.g., 12600 or 13010).

The tissue interface layer 14012 may or may not be used with any of the embodiments described herein. The tissue interface layer 14012 may have a central opening positioned beneath at least a portion of the gel material or gel layer 14006 as shown in Figure 12D. The central opening may allow the gel material or gel layer 14006 to contact a wound. The tissue interface layer 14012 may be adhered to a peripheral portion of the cover layer 14002. The tissue interface layer 14012 may assist in securing the perforated absorbent layer 14014 to the absorbent layer 14004. The tissue interface layer 14012 may comprise silicone.

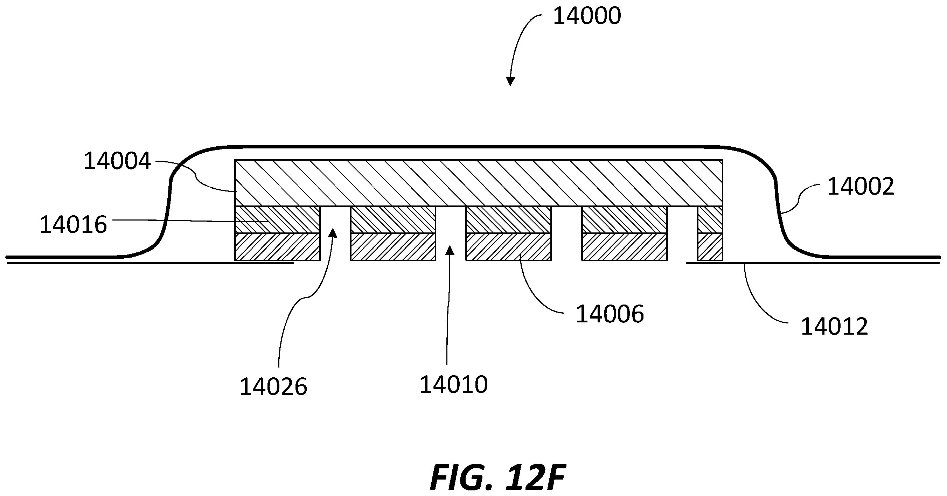

When the gel material or gel layer 14006 is applied to the perforated absorbent layer 14014 the perforations 14026 of the perforated absorbent layer 14014 should be avoided to reduce the chance of the gel material or gel layer 14006 filling the perforations of the perforated absorbent layer 14014. The gel material or gel layer 14006 may be cured quickly to prevent the gel material from permeating the perforated absorbent layer 14014 and causing the perforated absorbent layer 14014 to expand or swell and close the perforations 14026. The gel material or gel layer 14006 may form the gel perforations 14010. In some embodiments, for example, as discussed with reference to Figure 12F herein, it may be beneficial to allow the gel material or gel layer 14006 to permeate the absorbent layer 14004 or the perforated absorbent layer 14014 (e.g., creating gel permeated perforated absorbent layer 14016).

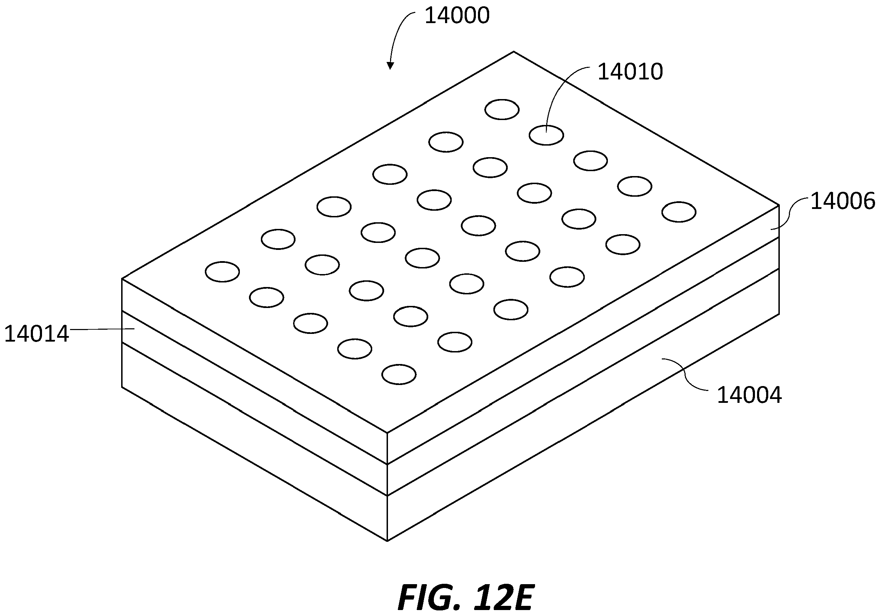

Figure 12E shows a bottom perspective view of a wound dressing 14000 having the perforated gel material or gel layer 14006, the perforated absorbent layer 14014, and the absorbent layer 14004. The tissue interface layer 14012 and the cover layer 14002 are not shown. As shown in Figure 12E, the gel material or gel layer 14006 has perforations 14010. As described herein, the perforations 14010 may be patterned. For example, the perforations 14010 may be arranged in lines with the perforations 14010 equally spaced and positioned next to each other. In some embodiments, the perforations 14010 may be positioned in a checkerboard type pattern with parallel lines and rows of perforations, or perforations maybe offset or staggered relative to each neighboring perforation 14010. The perforations 14010 may have any shape outer perimeter, for example, circular, square, rectangular, triangular, diamond, etc. The perforations 14010 may have different dimensions as they extend through the material or layer. For example, the perforations 14010 may have an overall pyramid shape. Described another way the perforations 14010 may increase or decrease in cross-sectional area as they extend through the layer or material.

The perforated absorbent layer 14014 may have perforations 14026 (e.g., as shown in Figure 12D). The perforations 14026 of the perforated absorbent layer 14014 may align with the perforations 14010 of the gel material or gel layer 14006. The perforations 14026 of the perforated absorbent layer 14014 may extend through the entire perforated absorbent layer 14014 until the perforations 14026 reach the absorbent layer 14004.

Figure 12F shows another embodiment of a wound dressing 14000. The wound dressing 14000 may include the cover layer 14002, the absorbent layer 14004, a gel permeated perforated absorbent layer 14016, the gel material or gel layer 14006, and the tissue interface layer 14012. The cover layer 14002 may be placed above the absorbent layer 14004. The gel permeated perforated absorbent layer 14016 may be placed below the absorbent layer 14004. The gel material or gel layer 14006 may be adhered to the wound-facing surface of the gel permeated perforated absorbent layer 14016. The optional tissue interface layer 14012 may be placed below the gel material or gel layer 14006 and adhered to a peripheral portion of the cover layer 14002.

The wound dressing 14000 of Figure 12F may include one or more nitrite providing layers (e.g., 12600 or 13010). In some embodiments, the wound dressing 14000 may include an acquisition distribution layer (e.g., 13008). The nitrite providing layer(s) (e.g., 12600 or 13010) may be positioned above the absorbent layer 14004, between the absorbent layer 14004 and of the gel permeated perforated absorbent layer 14016, between the gel material or gel layer 14006 and of the gel permeated perforated absorbent layer 14016, beneath the gel material or gel layer 14006, beneath the cover layer 14002, or between the cover layer 14002 and the absorbent layer 14004. In embodiments having an acquisition distribution layer (e.g., 13008), the acquisition distribution layer may be placed between the one or more nitrite providing layers and the gel material or gel layer 14006, between the cover layer 14002 and the absorbent layer 14004 or between the absorbent layer 14004 and the gel material or gel layer 14006.

In some embodiments the nitrite providing layer (e.g., 12600 or 13010) may be prepared and provided separate from the wound dressing 14000. For example, the nitrite providing layer (e.g., 12600 or 13010) may be applied to the wound and the wound dressing 14000 may be applied above the nitrite providing layer (e.g., 12600 or 13010).

In manufacturing the wound dressing 14000, a gel material may be permeated into a perforated absorbent layer to form the gel permeated perforated absorbent layer 14016. The gel permeated perforated absorbent layer 14016 may then be cured, for example, by using UV light or heat. The gel material or gel layer 14006 may then be adhered to the gel permeated perforated absorbent layer 14016. The size of the perforations 14026 in the gel permeated perforated absorbent layer 14016 may be sized to account for any swelling or expanding caused by permeating the gel material into the perforated absorbent layer.

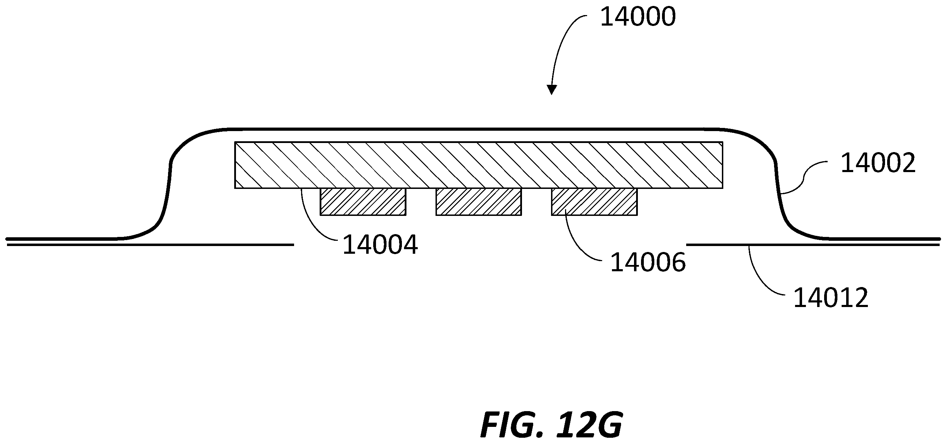

Figure 12G shows another embodiment of the wound dressing 14000. The wound dressing 14000 may have the cover layer 14002, the absorbent layer 14004, the gel material or gel layer 14006, and the tissue interface layer 14012. The wound dressing 14000 of Figure 12G may include one or more nitrite providing layers (e.g., 12600 or 13010). In some embodiments, the wound dressing 14000 may include an acquisition distribution layer (e.g., 13008). The nitrite providing layer(s) (e.g., 12600 or 13010) may be positioned above the absorbent layer 14004, between the gel material or gel layer 14006 and of the absorbent layer 14004, beneath the gel material or gel layer 14006, beneath the cover layer 14002, or between the cover layer 14002 and the absorbent layer 14004. In embodiments having an acquisition distribution layer (e.g., 13008), the acquisition distribution layer may be placed between the one or more nitrite providing layers and the gel material or gel layer 14006, between the cover layer 14002 and the absorbent layer 14004 or between the absorbent layer 14004 and the gel material or gel layer 14006.

In some embodiments the nitrite providing layer (e.g., 12600 or 13010) may be prepared and provided separate from the wound dressing 14000. For example, the nitrite providing layer (e.g., 12600 or 13010) may be applied to the wound and the wound dressing 14000 may be applied above the nitrite providing layer (e.g., 12600 or 13010).

In some embodiments, the gel material or gel layer 14006 may only be adhered to a portion of the absorbent layer 14004. The gel material or gel layer 14006 may be adhered to the absorbent layer 14004 by applying a mold with openings to the absorbent layer 14004. The openings of the mold can be any shape, for example, circular, square, diamonds, pyramids, etc. The mold may be attached or clamped to the absorbent layer 14004. The gel material or gel layer 14006 may be applied to the absorbent layer 14004 through the openings of the mold. In some methods, each individual hole of the mold may be filled one by one. In some methods, the entire mold may be filled at once. Once applied, the gel material or gel layer 14006 may be cured.

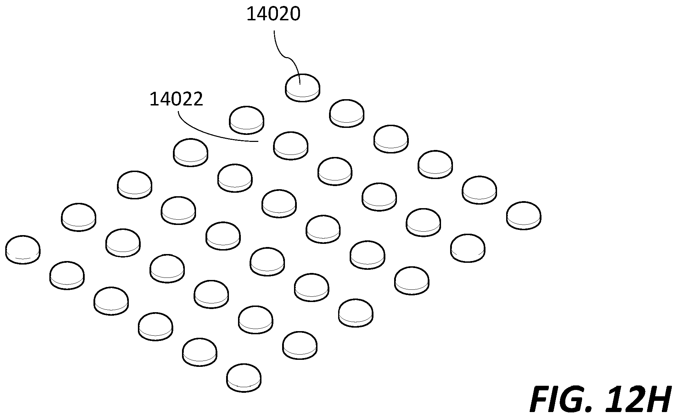

The use of a mold with openings may form the gel material 1406 into gel formations 14020 as shown in Figure 12H. The gel formations 14020 may be formed with gaps 14022 between them. The gaps 14022 may act as fluid channels and allow for fluid passage to the absorbent layer 14004. The gel formations 14020 may have shapes that correspond to the shapes of the openings of the mold, for example, circular, square, diamonds, pyramids, etc.

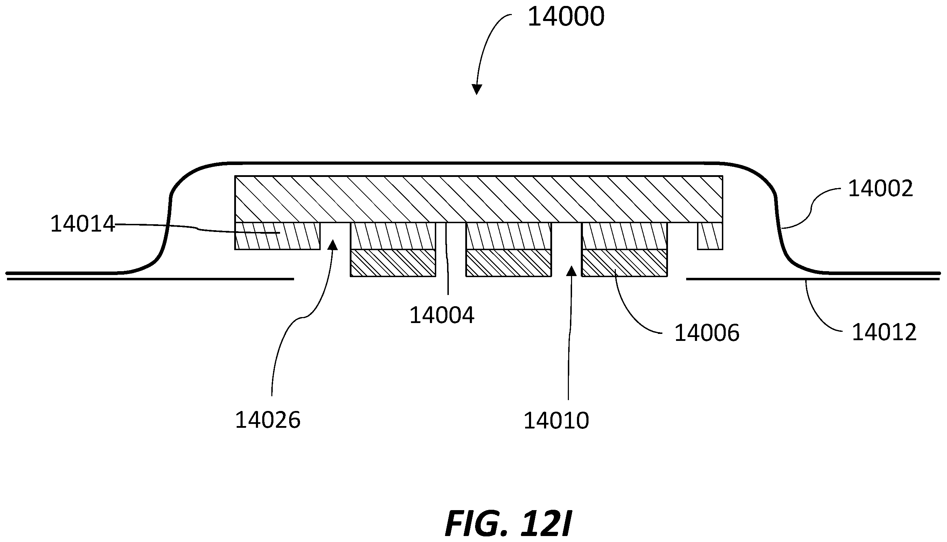

Figure 12I depicts another embodiment of the wound dressing 14000. The wound dressing 14000 may include the cover layer 14002, the absorbent layer 14004, the perforated absorbent layer 14014, the gel material, and the tissue interface layer 14012. The absorbent layer 14004 may be placed below the cover layer 14002. The perforated absorbent layer 14014 may be placed below the absorbent layer 14004. The gel material or gel layer 14006 may be adhered to the perforated absorbent layer 14004. In some embodiments, the gel material or gel layer 14006 may be permeated into the perforated absorbent layer 14004 to create a gel permeated perforated absorbent layer 14016 as described with reference to Figure 12F. The wound dressing 14000 of Figure 12I may include one or more nitrite providing layers (e.g., 12600 or 13010). In some embodiments, the wound dressing 14000 may include an acquisition distribution layer (e.g., 13008). The nitrite providing layer(s) (e.g., 12600 or 13010) may be positioned above the absorbent layer 14004, between the gel material or gel layer 14006 and of the absorbent layer 14004, beneath the gel material or gel layer 14006, beneath the cover layer 14002, or between the cover layer 14002 and the absorbent layer 14004. The nitrite providing layer(s) (e.g., 12600 or 13010) may be positioned above or below the perforated absorbent layer 14004.

In some embodiments the nitrite providing layer (e.g., 12600 or 13010) may be prepared and provided separate from the wound dressing 14000. For example, the nitrite providing layer (e.g., 12600 or 13010) may be applied to the wound and the wound dressing 14000 may be applied above the nitrite providing layer (e.g., 12600 or 13010).

In some embodiments, the gel material or gel layer 14006 may be adhered to only a portion of the perforated absorbent layer 14014 as shown in Figure 12I. The tissue interface layer 14012 may be placed below the gel material or gel layer 14006 be adhered to a peripheral portion of the cover layer 14002 and have an opening that corresponds to the portion of the perforated absorbent layer 14014 having a gel material or gel layer 14006 adhered to it. The tissue interface layer 14012 may assist in securing the perforated absorbent layer 14014 to the absorbent layer 14014.

During manufacturing, a constraining ring or clamp, can be applied to the perforated absorbent layer 14014. The constraining ring or clamp can prevent the gel material or gel layer 14006 from adhering to the entire wound-facing surface of the perforated absorbent layer 14014. The size and shape of the constraining ring or clamp may determine the surface area of the perforated absorbent layer 14014 that the gel material or gel layer 14006 is cured to. The gel material or gel layer 14006 may be applied to the perforated absorbent layer 14014 while the constraining ring or clamp is applied and then cured, for example, via UV light or heat. The constraining ring or clamp may then be removed. The perforated absorbent layer 14014 may then be connected to the absorbent layer 14004.

Samples of some of the embodiments described were manufactured using a hydrogel monomer solution (i.e., 0.7 SA with 1.4% sodium isoascorbate) with approximately 2.5 g applied to a 10.8 cm x 10.8 cm square of a 17 gsm pre-perforated or non-perforated mesh interleave. In perforated samples, perforations having diameters of 3 mm, 4.5 mm, and 6 mm were tested. In one specific example. the saturated interleave was then placed onto a 10.8 cm x 10.8 cm square of ALLEVYN^{™} foam, 4 mm thick, and cured. ALLEVYN^{™} foam samples of varying sizes were tested including 5 cm x 5 cm samples and 10 cm x 10 cm samples. A top film or cover layer was applied above the ALLEVYN^{™} foam. A nitrite providing layer was applied to a simulated wound and then the ALLEVYN^{™} foam layer with the cured mesh interleave was applied on top of the nitrite providing layer. These samples were found to successfully release nitric oxide while absorbing fluid into the dressing. The described testing is exemplary and does not limit the embodiments described herein.

### Embodiments with Composite ADL

Any of the embodiments or aspects described herein may be incorporated into a wound dressing that utilizes an acquisition distribution layer and/or an additional activator layer, as described above. In some embodiments, any of the embodiments described herein may be incorporated into a wound dressing that comprises a composite acquisition distribution layer. In embodiments having a composite acquisition distribution layer and a gel containing wound contact layer (e.g., as described with reference to Figures 12A-I), both the gel containing wound contact layer and the composite acquisition distribution layer can provide acid to the nitrite providing layer. The composite acquisition distribution layer is described in more detail in Appendix A, which should be considered to be a part of this specification. The absorbent layers and gel wound contact layers described herein, may be added or substituted as a wound contact layer in the embodiments described in Appendix A, for example below the layers of the dressings described in Appendix A.

### Terminology

Features, materials, characteristics, or groups described in conjunction with a particular aspect, embodiment, or example are to be understood to be applicable to any other aspect, embodiment or example described herein unless incompatible therewith. All of the features disclosed in this specification (including any accompanying claims, abstract and drawings), or all of the steps of any method or process so disclosed, may be combined in any combination, except combinations where at least some of such features or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

While certain embodiments have been described, these embodiments have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure. Depending on the embodiment, certain of the steps described above may be removed, others may be added. Furthermore, the features and attributes of the specific embodiments disclosed above may be combined in different ways to form additional embodiments, all of which fall within the scope of the present disclosure.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Likewise, the term "and/or" in reference to a list of two or more items, covers all of the following interpretations of the word: any one of the items in the list, all of the items in the list, and any combination of the items in the list. Further, the term "each," as used herein, in addition to having its ordinary meaning, can mean any subset of a set of elements to which the term "each" is applied. Additionally, the words "herein," "above," "below," and words of similar import, when used in this application, refer to this application as a whole and not to any particular portions of this application.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

Any of the embodiments described herein can be used with a canister or without a canister. Any of the dressing embodiments described herein can absorb and store wound exudate.

## Claims

1. A wound dressing apparatus, comprising:
an absorbent layer having a wound-facing surface and an opposite, non-wound facing surface;
a gel material adhered to the wound-facing surface of the absorbent layer, wherein the gel material comprises a cured patterned structure defining a plurality of fluid channels extending therethrough, wherein the gel material comprises acidic groups or moieties,
a cover layer positioned above the absorbent layer and
one or more nitrite providing layers, wherein the one or more nitrite providing layers is positioned beneath the cover layer.

2. The wound dressing apparatus of Claim 1, further comprising a tissue interface layer below the gel material and adhered to a peripheral portion of the cover layer, wherein the tissue interface layer comprises a central opening positioned beneath at least a portion of the gel material; optionally, wherein the tissue interface layer comprises silicone.

3. The wound dressing apparatus of Claim 1 or Claim 2, wherein the one or more nitrite providing layers is positioned beneath the gel material, or is positioned between the cover layer and the absorbent layer.

4. The wound dressing apparatus of any one of Claims 1 to 3, further comprising an acquisition distribution layer.

5. The wound dressing apparatus of Claim 4, wherein the acquisition distribution layer is a composite material comprising a gel material comprising acidic groups or moieties.

6. The wound dressing apparatus of any one of Claims 1 to 5, wherein the gel material adhered to the wound-facing surface of the absorbent layer is configured to provide acid to the one or more nitrite providing layers to release nitric oxide.

7. The wound dressing apparatus of any one of the preceding claims, wherein the gel material is provided on a mesh.

8. The wound dressing apparatus of any one of the preceding claims, wherein the absorbent layer is perforated.

9. The wound dressing apparatus of any one of the preceding claims, wherein the gel material is at least partially saturated into the absorbent layer.

10. The wound dressing apparatus of Claim 8, wherein the perforated absorbent layer is saturated with the gel material and further comprising a second gel material adhered to the perforated absorbent layer.

11. The wound dressing apparatus of any one of the preceding claims, wherein the cured pattern structure has a smaller outer perimeter than an outer perimeter of the absorbent layer.

## Patentansprüche

1. Wundverbandvorrichtung umfassend:
eine Absorptionsschicht mit einer wundzugewandten Oberfläche und einer gegenüberliegenden, nicht wundzugewandten Oberfläche;
ein Gelmaterial, das an der wundzugewandten Oberfläche der Absorptionsschicht haftet, wobei das Gelmaterial eine gehärtete strukturierte Struktur umfasst, die eine Vielzahl von Fluidkanälen definiert, die durch diese hindurch verlaufen, wobei das Gelmaterial saure Gruppen oder Einheiten umfasst,
eine Deckschicht, die über der Absorptionsschicht angeordnet ist, und
eine oder mehrere Nitrit-bereitstellende Schichten, wobei die eine oder mehreren Nitrit-bereitstellenden Schichten unter der Deckschicht angeordnet sind.

2. Wundverbandvorrichtung nach Anspruch 1, ferner umfassend eine Gewebegrenzflächenschicht unter dem Gelmaterial und an einem peripheren Abschnitt der Deckschicht haftend, wobei die Gewebegrenzflächenschicht eine zentrale Öffnung umfasst, die unter wenigstens einem Abschnitt des Gelmaterials angeordnet ist; wobei gegebenenfalls die Gewebegrenzflächenschicht Silikon umfasst.

3. Wundverbandvorrichtung nach Anspruch 1 oder Anspruch 2, wobei die eine oder mehreren Nitrit-bereitstellenden Schichten unter dem Gelmaterial angeordnet sind oder zwischen der Deckschicht und der Absorptionsschicht angeordnet sind.

4. Wundverbandvorrichtung nach einem der Ansprüche 1 bis 3, ferner umfassend eine Erfassungsverteilungsschicht.

5. Wundverbandvorrichtung nach Anspruch 4, wobei die Erfassungsverteilungsschicht ein Verbundmaterial ist, das ein Gelmaterial umfassend saure Gruppen oder Einheiten umfasst.

6. Wundverbandvorrichtung nach einem der Ansprüche 1 bis 5, wobei das Gelmaterial, das an der wundzugewandten Oberfläche der Absorptionsschicht haftet, dafür gestaltet ist, der einen oder den mehreren Nitrit-bereitstellenden Schichten Säure bereitzustellen, um Stickoxid freizusetzen.

7. Wundverbandvorrichtung nach einem der vorstehenden Ansprüche, wobei das Gelmaterial auf einem Netz bereitgestellt ist.

8. Wundverbandvorrichtung nach einem der vorstehenden Ansprüche, wobei die Absorptionsschicht perforiert ist.

9. Wundverbandvorrichtung nach einem der vorstehenden Ansprüche, wobei das Gelmaterial wenigstens teilweise in die Absorptionsschicht gesättigt ist.

10. Wundverbandvorrichtung nach Anspruch 8, wobei die perforierte Absorptionsschicht mit dem Gelmaterial gesättigt ist und ferner ein zweites Gelmaterial umfasst, das an der perforierten Absorptionsschicht haftet.

11. Wundverbandvorrichtung nach einem der vorstehenden Ansprüche, wobei die gehärtete strukturierte Struktur einen kleineren Außenumfang als ein Außenumfang der Absorptionsschicht aufweist.

## Revendications

1. Appareil pour pansements, comprenant :
une couche absorbante ayant une surface côté plaie et une surface opposée, ne faisant pas face à la plaie ;
un matériau gélifié adhérant à la surface côté plaie de la couche absorbante, le matériau gélifié comprenant une structure à motif durcie définissant une pluralité de canaux fluidiques s'étendant à travers celle-ci, le matériau gélifié comprenant des groupes ou fractions acides ;
une couche de recouvrement positionnée au-dessus de la couche absorbante ; et
une ou plusieurs couches fournissant du nitrite, la ou les couches fournissant du nitrite étant positionnées en dessous de la couche de recouvrement.

2. Appareil pour pansements de la revendication 1, comprenant en outre une couche d'interface tissulaire sous le matériau gélifié et adhérant à une partie périphérique de la couche de recouvrement, la couche d'interface tissulaire comprenant une ouverture centrale positionnée en dessous d'au moins une partie du matériau gélifié, la couche d'interface tissulaire comprenant éventuellement du silicone.

3. Appareil pour pansements de la revendication 1 ou la revendication 2, dans lequel la ou les couches fournissant du nitrite sont positionnées en dessous du matériau gélifié, ou sont positionnées entre la couche de recouvrement et la couche absorbante.

4. Appareil pour pansements de l'une quelconque des revendications 1 à 3, comprenant en outre une couche d'acquisition-distribution.

5. Appareil pour pansements de la revendication 4, dans lequel la couche d'acquisition-distribution est un matériau composite comprenant un matériau gélifié comprenant des groupes ou fractions acides.

6. Appareil pour pansements de l'une quelconque des revendications 1 à 5, dans lequel le matériau gélifié adhérant à la surface côté plaie de la couche absorbante est conçu pour fournir de l'acide à la couche ou aux couches fournissant du nitrite pour libérer de l'oxyde nitrique.

7. Appareil pour pansements de l'une quelconque des revendications précédentes, dans lequel le matériau gélifié est disposé sur une toile.

8. Appareil pour pansements de l'une quelconque des revendications précédentes, dans lequel la couche absorbante est perforée.

9. Appareil pour pansements de l'une quelconque des revendications précédentes, dans lequel le matériau gélifié est au moins partiellement saturé à l'intérieur de la couche absorbante.

10. Appareil pour pansements de la revendication 8, dans lequel la couche absorbante perforée est saturée avec le matériau gélifié, et comprenant en outre un deuxième matériau gélifié adhérant à la couche absorbante perforée.

11. Appareil pour pansements de l'une quelconque des revendications précédentes, dans lequel la structure à motif durcie a un périmètre externe plus petit qu'un périmètre externe de la couche absorbante.
